# EUROPEAN PATENT SPECIFICATION

(11) **EP 4 196 196 B1**
(45) Date of publication and mention of the grant of the patent: **07.10.2026**
(21) Application number: 21856817.8
(22) Date of filing: 13.08.2021
(51) Int. Cl.: A61M 5/46, A61F 9/007, A61M 5/00, A61M 5/315

(54) **MOTORIZED INJECTION SYSTEM**
MOTORISIERTES INJEKTIONSSYSTEM
SYSTÈME D'INJECTION MOTORISÉ

(30) Priority: 13.08.2020 US 202063064975 P
(43) Date of publication of application: 21.06.2023
(73) Proprietor: MeiraGTx Ocular UK Limited, London N1 7NQ (GB)
(72) Inventor: LAULICHT, Bryan, New York, New York 10011 (US); CHITNIS, Girish, Watertown, Massachusetts 02472 (US); AHN, Edward, Dover, Massachusetts 02030 (US)
(74) Representative: CMS Cameron McKenna Nabarro Olswang LLP
(86) International application number: PCT/US2021/046001
(87) International publication number: WO 2022/036256

(56) References cited:
- WO-A1-2021/055906
- US-A1- 2006 122 555
- US-A1- 2015 182 700
- US-A1- 2015 182 700
- US-A1- 2020 069 883
- US-A1- 2020 069 883
- US-B1- 6 200 289
- US-B2- 7 025 774

## Description

### FIELD

The present disclosure is related to a system and method that enables an injection into a cavity or a void, and in particular through a tissue into a cavity or void in a human body, such as the suprachoroidal space in ocular tissue.

### BACKGROUND

The present disclosure is related to a device and method that enables delivery of multiple therapeutics to a cavity or a void of a human body, and in particular to ocular tissue in posterior segment of the eye through the suprachoroidal space. Posterior segment eye diseases are a major cause of permanent visual impairment affecting millions of people which can lead to blindness if left untreated. It includes multiple diseases such as age-related macular degeneration (AMD), diabetic retinopathy, diabetic macular edema (DME), choroidermeia (CHM), retinal vein occlusion (RVO), uveitis, and endophthalmitis. Although pharmaceutical agents may be available to prevent disease progression in many cases, systemic delivery cannot achieve therapeutic concentrations in the posterior segment due to blood-eye barrier.

Recently, the suprachoroidal space (SCS) has been explored as the potential drug delivery route to the back of the eye. The suprachoroidal space is the potential space between the sclera and the choroid. Drug delivered in this space can go around the eye globe to the posterior segment of the eye. This route for drug delivery has been shown to be more effective for treatment of the posterior segment than intravitreal injections. However, the simplicity of intravitreal injection outweighs the surgical procedure previously needed for suprachoroidal delivery. Historically, suprachoroidal delivery was achieved by creating small incision using scalpel, followed by delivery using a needle or cannula. More recently, a microneedle with a predefined, short length, which allows penetration only up to certain depth, has been used to target suprachoroidal space. Because the scleral thickness varies significantly within the patient populations, either prior mapping of eye geometry, or trial and error, is necessary while injecting with hollow microneedles. If the needle is too long, it can easily penetrate through the thin suprachoroidal space to inject the drug in the vitreous; and, if it is too short, it delivers into the sclera. The sclera is 10 times stiffer than the choroid and 200 times stiffer than the retina making it even more challenging to pierce the sclera without injecting into the vitreous. In some instances, a small volume (on the order of 100 microliters) of therapeutic needs to be injected into the suprachoroidal space, and it needs to be injected with sufficient force to displace the positive resistance of intraocular pressure pressing the choroid against the sclera to achieve a broad coverage of the posterior segment of the eye. This may be difficult to achieve using a conventional hand-held syringe.

Accordingly, there is a need for an improved system and method for suprachoroidal drug delivery that precisely, consistently and safely targets the suprachoroidal space and provides broad coverage of the posterior segment of the eye.
US 2020/069883 A1 discloses a system for delivering an injection of a fluid into a void within a subject. A barrel extends from a first end to a second end and forms a lumen extending from the first end to the second end. A plug and a floating seal are arranged within the lumen. A hollow needle includes a distal end with an opening for fluid to flow from the lumen. The plug, the barrel and the floating seal include material and dimensions selected based on a threshold flowrate for a fluid arranged within the lumen to overcome a force opposed to a force being applied to the fluid in the lumen to move the floating seal and the hollow needle into a tissue of the subject, and succumb to the opposing force to displace the fluid through the opening into the void.
US 2006/0122555 A1 discloses an automatic injection device which includes a drive mechanism and a sensor used lo determine an internal characteristic such as a force or internal pressure generated during an injection process. This characteristic is then used as a control parameter by a microprocessor or controller to determine the exit pressure of the fluid expelled by the device. This exit pressure is then used to identify the kind of tissue in which the injection is being introduced.
US 2015/0182700 A1 discloses a system that senses the pressure of a suction cap and automatically performs injection. The system of US 2015/0182700 A1 comprises a suction motor for increasing the suction pressure to the suction cap of a suction injection needle; a solenoid valve having a solenoid valve controller connected thereto and having the function of relieving the suction pressure of the suction cap; a pressure sensor having a drug injection controller connected thereto and measuring the pressure of the suction cap; a drug injection motor operated by the drug injection controller on the basis of the pressure of the suction injection needle measured by the pressure sensor; a screw gear bolt connected to a syringe piston pusher which pushes a syringe piston along the lengthwise direction of a drug injection supporting portion; a screw gear shaft passing through the screw gear bolt and rotating according to the operation of the drug injection motor; and a connecting member for interconnecting the solenoid valve, the suction motor, the pressure sensor, and the suction injection needle. Thus, after the suction cap is attached to the skin, the pressure is sensed and a set amount of drugs is automatically injected.

### SUMMARY

According to some aspects of the present disclosure, there is provided an injection system comprising an injection assembly comprising a syringe barrel defining a lumen between a proximal end and a distal end and a second sealing element moveably disposed within the lumen to dispense an injection agent from an injection chamber defined in the syringe barrel, and a puncture element configured to deliver the injection agent into a space in a tissue of a patient. The tissue is less permeable to the injection agent than the space. The injection system also includes a support platform configured to support the injection assembly and anchor the injection assembly relative to a site of injection, a drive assembly configured to operate the injection assembly, one or more sensors configured to monitor one or more forces on the injection assembly, and a controller in communication with the one or more sensors to receive information about the one or more forces on the injection system. The controller is configured, based on the information, to operate the drive assembly to advance the puncture element through the tissue toward the space such that the injection agent remains in the injection chamber until the puncture element fluidly connects the injection chamber with the space.

In some embodiments, the injection assembly further includes a first sealing element moveably disposed within the lumen distal to the second sealing element, wherein the first sealing element and the second sealing element form a seal with the lumen and define the injection chamber between them. The puncture element can be in fluid communication with the injection chamber to deliver the injection agent from the injection chamber into a space in a tissue of a patient. When a force is applied on the second sealing element in a distal direction, in response to a first opposing force as the puncture element advances through the tissue, the first sealing element moves in the distal direction to advance the puncture element in the distal direction, without conveying the injection agent through the puncture element, and in response to a second opposing force when the injection chamber is fluidly connected to the space, the first sealing element remains stationary and the injection agent is conveyed from the injection chamber through the puncture element.

In some embodiments, the drive assembly is linked to the second sealing element to apply the force on the second sealing element to translate the second element in the distal direction. In some embodiments, the drive assembly comprises a linear actuator linked the second sealing element to apply the force on the second sealing element to translate the second element in the distal direction. In some embodiments, the drive assembly comprises a first driver configured to translate the syringe barrel relative to the support platform and a second driver linked to the second sealing element to translate the second sealing element relative to the syringe barrel. The one or more sensors can comprise a first load cell configured to measure force on the syringe barrel, and the one or more sensors can comprise a second load cell configured to measure force on the second sealing element. In some embodiments, the one or more sensors comprise one or more of a pressure sensor, force sensor, strain sensor, position sensor or low rate sensor.

In some embodiments, the controller is programmed to implement one or more feedback loops to monitor the first opposing force and the second opposing force. In some embodiments, the controller is programmed to implement one or more feedback loops to monitor a pre-insertion of the puncture element into the tissue, with the one or more feedback loops being configured to monitor an increase in force onto the puncture element, to detect a drop in force on the puncture element, and based on the drop, to cause an advancement of the puncture element by a predetermined distance to imbed the puncture element into the tissue. In some embodiments, the controller is programmed to implement one or more feedback loops to monitor an advancement of the puncture element through the tissue, the one or more feedback loops being configured to measure a load on the second sealing element and detect a drop in the load once the puncture element reaches the space in the tissue. In some embodiments, the controller is programmed to implement one or more feedback loops to monitor an injection of the injection agent into the space, with the one or more feedback loops being configured to control a velocity or an advancement distance of the second sealing element. In some embodiments, the controller is programmed to cause a retraction of the puncture element by a pre-determined distance when the one or more sensors detect a drop in load on the second sealing element. In some embodiments, the controller is programmed to control a stopping distance of the puncture element as the puncture element enters the space.

In some embodiments, the tissue is conjunctiva and the space is subconjunctival space. In some embodiments, the tissue is sclera and the space is suprachoroidal space. In some embodiments, the tissue is sclera and choroid and the space is intravitreal space. In some embodiments, the tissue is cornea and the space is an anterior chamber of an eye.

In some aspects, the present disclosure provides an injection system that comprises an injection assembly comprising a syringe barrel defining a lumen between a proximal end and a distal end, a first sealing element and a second sealing element moveably disposed within the lumen. The second sealing element is distal to the first sealing element to define an injection chamber. A puncture element is fluidly connected to the injection chamber and configured to deliver an injection agent from the injection chamber into a space in a tissue of a patient. The tissue is less permeable to the injection agent than the space. The injection system also includes a support platform configured to support the injection assembly and anchor the injection assembly relative to a site of injection, a drive assembly configured to translate one or both of the syringe barrel or the second sealing element relative to the support platform, one or more sensors configured to monitor one or more forces on the injection assembly, and a controller in communication with the one or more sensors to receive information about the one or more forces on the injection system. The controller in communication with the one or more sensors receives information about the one or more forces on the injection system, and is configured to, based on the information, to control the drive assembly to advance the puncture element through the tissue toward the space such that when the drive assembly translates the second sealing element in a distal direction. In response to a first opposing force as the puncture element advances through the tissue, the first sealing element moves in the distal direction to advance the puncture element in the distal direction, without conveying the injection agent through the puncture element, and in response to a second opposing force when the injection chamber is fluidly connected to the space, the first sealing element remains stationary and the injection agent is conveyed from the injection chamber through the puncture element.

In some embodiments, the drive assembly is configured to translate independently of one another the syringe barrel and the second sealing element relative to the support platform. In some embodiments, the drive assembly is linked to the second sealing element to apply the force on the second sealing element to translate the second element in the distal direction. In some embodiments, the drive assembly comprises a linear actuator linked the second sealing element to apply the force on the second sealing element to translate the second element in the distal direction. In some embodiments, the drive assembly comprises a first driver configured to translate the syringe barrel relative to the support platform and a second driver linked to the second sealing element to translate the second sealing element relative to the syringe barrel.

In some embodiments, the one or more sensors comprise a first load cell configured to measure force on the syringe barrel. In some embodiments, the one or more sensors comprise a second load cell configured to measure force on the second sealing element. In some embodiments, the one or more sensors comprise one or more of a pressure sensor, force sensor, strain sensor, position sensor or low rate sensor.

In some embodiments, the controller is programmed to implement one or more feedback loops to monitor the first opposing force and the second opposing force. In some embodiments, the controller is programmed to implement one or more feedback loops to monitor a pre-insertion of the puncture element into the tissue. The one or more feedback loops can be configured to monitor an increase in force onto the puncture element, to detect a drop in force on the puncture element, and based on the drop, to cause an advancement of the puncture member by a predetermined distance to imbed the puncture element into the tissue. In some embodiments, the controller is programmed to implement one or more feedback loops to monitor an advancement of the puncture element through the tissue, with the one or more feedback loops being configured to measure a load on the second sealing element and detect a drop in the load once the puncture element reaches the space in the tissue. In some embodiments, the controller is programmed to implement one or more feedback loops to monitor an injection of the injection agent into the space, with the one or more feedback loops being configured to control a velocity or an advancement distance of the second sealing element. In some embodiments, the controller is programmed to cause a retraction of the puncture element by a pre-determined distance when the one or more sensors detect a drop in load on the second sealing element. In some embodiments, the controller is programmed to control a stopping distance of the puncture element as the puncture element enters the space.

In some embodiments, the tissue is conjunctiva and the space is subconjunctival space. In some embodiments, the tissue is sclera and the space is suprachoroidal space. In some embodiments, the tissue is sclera and choroid and the space is intravitreal space. In some embodiments, the tissue is cornea and the space is an anterior chamber of an eye.

A method of delivering an injection agent is provided that comprises inserting a puncture element into a tissue. The puncture element can be configured to deliver an injection agent from an injection chamber into a space in the tissue, with the tissue having a density greater than the space such that the tissue is less permeable to the injection agent than the space. The method also includes advancing, using a drive assembly, the puncture element through the tissue toward the space, monitoring one or more forces on the puncture element using one or more sensors, and controlling, using a controller in communication with the one or more sensors, the drive assembly to advance the puncture element through the tissue toward the space such that the injection agent remains in the injection chamber until the puncture element fluidly connects the injection chamber with the space.

In some embodiments, the puncture element is positioned on a distal end of an injection assembly comprising a syringe barrel defining a lumen between a proximal end and a distal end and a first and second sealing element moveably disposed within the lumen to dispense the injection agent from the injection chamber. In some embodiments, in response to a first opposing force of the one or more force on the puncture element as it advances through the tissue, the first sealing element moves in a distal direction to advance the puncture element in the distal direction, without conveying the injection agent through the puncture element. In some embodiments, in response to a second opposing force of the one or more forces on the puncture element when the injection chamber is fluidly connected to the space, the first sealing element remains stationary and the second seal element moves in a distal direction such that the injection agent is conveyed from the injection chamber through the puncture element into the space.

In some embodiments, the tissue is conjunctiva and the space is subconjunctival space. In some embodiments, the tissue is sclera and the space is suprachoroidal space. In some embodiments, the tissue is sclera and choroid and the space is intravitreal space. In some embodiments, the tissue is cornea and the space is an anterior chamber of an eye.

A method of delivering an injection agent is provided that comprises positioning an injection assembly adjacent a tissue. The injection assembly comprises a syringe barrel defining a lumen between a proximal end and a distal end and a second sealing element moveably disposed within the lumen to dispense an injection agent from an injection chamber defined in the syringe barrel, and a puncture element extending configured to deliver the injection agent into a space in the tissue. The tissue has a density greater than the space such that the tissue is less permeable to the injection agent than the space. The method also includes monitoring one or more forces on the injection assembly using one or more sensors, and controlling the injection assembly using the forces on the injection system using a controller in communication with the one or more sensors to advance the puncture element through the tissue toward the space such that the injection agent remains in the injection chamber until the puncture element fluidly connects the injection chamber with the space.

In some embodiments, in response to a first opposing force of the one or more force on the puncture element as it advances through the tissue, the first sealing element moves in a distal direction to advance the puncture element in the distal direction, without conveying the injection agent through the puncture element. In some embodiments, in response to a second opposing force of the one or more forces on the puncture element when the injection chamber is fluidly connected to the space, the first sealing element remains stationary and the second seal element moves in a distal direction such that the injection agent is conveyed from the injection chamber through the puncture element into the space. In some embodiments, the method further comprises anchoring the injection assembly relative to a site of injection in the tissue.

In some embodiments, the tissue is conjunctiva and the space is subconjunctival space. In some embodiments, the tissue is sclera and the space is suprachoroidal space. In some embodiments, the tissue is sclera and choroid and the space is intravitreal space. In some embodiments, the tissue is cornea and the space is an anterior chamber of an eye.

### BRIEF DESCRIPTION OF THE DRAWINGS

The present disclosure is further described in the detailed description which follows, in reference to the noted plurality of drawings by way of non-limiting examples of exemplary embodiments, in which like reference numerals represent similar parts throughout the several views of the drawings, and wherein
FIG. 1A illustrates an exemplary graph of time (or displacement) versus force to show forces experienced by a motorized injection system when administering a therapeutic agent into a tissue;
FIG. 1B illustrates an exemplary graph of piston position versus applied force to show forces experienced by a motorized injection system when administering a therapeutic agent into a tissue;
FIG. 2 illustrates an exemplary embodiment of a motorized injection system;
FIG. 3A and FIG. 3B illustrate an exemplary embodiment of a drive assembly for a syringe barrel and a syringe plunger;
FIG. 4 illustrates an exemplary embodiment of means for attaching and stabilizing a motorized injection system to an injection site;
FIG. 5A illustrates an exemplary embodiment of an injection system;
FIG. 5B illustrates an exemplary method of use of an embodiment of the injection system of FIG. 5A;
FIGS. 6A, 6B, 6C, and 6D illustrate an embodiment of a use of a motorized injection system with an auto-stop syringe;
FIGS. 7A and 7B are a flowchart showing the use of the system shown in FIGS. 6A-6D;
FIGS. 8A, 8B, 8C, 8D, and 8E illustrate an embodiment of a use of a motorized injection system with an auto-stop syringe;
FIGS. 9A and 9B are a flowchart showing the use of the system shown in FIGS. 8A-8E;
FIGS. 10A, 10B, 10C, 10D, and 10E illustrate an embodiment of a use of a motorized injection system with an auto-stop syringe;
FIGS. 11A and 11B are a flowchart showing the use of the system shown in FIGS. 10A-10E;
FIG. 12 shows an embodiment of an injection system of the present disclosure having a rapid fill port;
FIGS. 13A-13B and 14A-14B illustrate an exemplary process of filling an injection system of the present disclosure through a rapid fill port;
FIGS. 15A-15B show an exemplary process of back filling an injection system of the present disclosure;
FIGS. 16A-16B show an exemplary process of filling an injection system of the present disclosure through a port in the proximal end;
FIGS. 17A-17C show an exemplary process of filling an injection system of the present disclosure through a port sealed with a self-sealing polymer;
FIGS. 18A-18D show embodiments of an injection system of the present disclosure having a port in the distal end; and
FIG. 19 is an exemplary embodiment of a computing system for use with various embodiments of the present disclosure.

While the above-identified drawings set forth presently disclosed embodiments, other embodiments are also contemplated, as noted in the discussion. This disclosure presents illustrative embodiments by way of representation and not limitation.

### DETAILED DESCRIPTION

The present disclosure provides a motorized injection system for delivery of a therapeutic agent into a potential space or cavity in tissue. In some embodiments, such systems can be used for drug delivery to the suprachoroidal space. In some embodiments, such systems are automated and comprise of sensors and feedback loops. The systems of the present disclosure can thus be configured to precisely, consistently and safely target the suprachoroidal space and to provide broad coverage of the posterior segment of the eye.

In some embodiments, the injection system comprises a syringe barrel for holding one or more injection agents, a penetrating element (also referred as needle, but similar devices can be used) attached to the syringe barrel in fluid communication with the syringe barrel and a sealing element (also referred as a pushing plunger) to expel injection agent from the syringe barrel through the needle. As described in more detail below, the injection system can include a regular syringe or an auto-stop syringe with multiple sealing elements as described in more detail below.

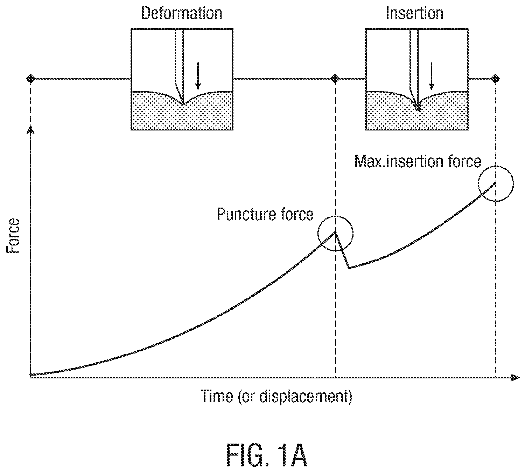

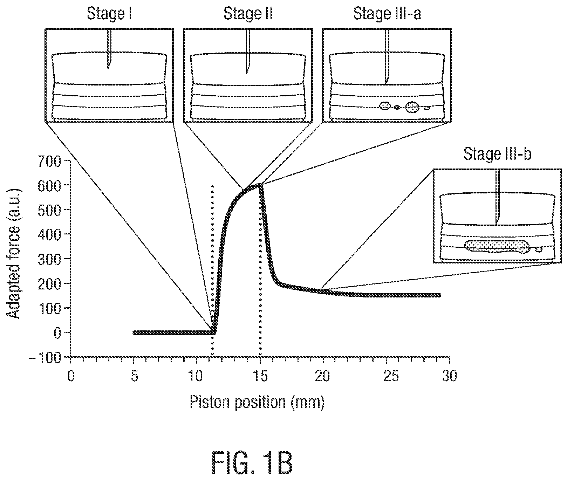

FIG. 1A and FIG. 1B illustrate the forces experienced by the motorized injection system when administering a therapeutic agent into a tissue cavity through a puncture member or element (referred to interchangeably as a needle but a similar device can also be used). At Stage I, the needle is pre-inserted into the tissue (for example, sclera of the eye). In reference to FIG. 1A, during pre-insertion of the needle into the tissue (movement of the injection system towards the tissue), the load cell attached to the injection system registers an increasing force until the needle punctures the tissue. When the tissue is punctured, there may be a drop in the load. In reference to FIG. 1B, which shows the applied load after the completion of Stage I, once the insertion is complete, the contents of the injection system can be pressurized by advancing a pushing plunger. The load on the plunger may remain constant at this stage as the plunger advances until the pressure in the syringe barrel starts to increase. Next, at Stage II, the needle is advanced through the tissue toward a cavity (for example, the suprachoroidal space of the eye). At this stage, due to the low water permeability of the tissue, the contents of the injection system remain pressurized. The load on the injection system increases and may plateau. At Stage III-a, the needle tip enters the cavity (for example, the suprachoroidal space of the eye). Because the density of the cavity is less than the density of the tissue, the cavity produces less backpressure against the needle than the backpressure produced by the tissue. Accordingly, once the lumen of the needle opens up into the cavity, the load on the plunger falls due to the decrease in the backpressure. This drop in the backpressure signals that the needle lumen is in the cavity, and the needle can be prevented from advancing any deeper into the cavity (either by the system or due to the auto-adjustment design as discussed below). In some embodiments, the therapeutic agent can be pressurized to a pressure that is insufficient to expel the therapeutic agent into the tissue, but is sufficient to expel the therapeutic agent into the cavity. Accordingly, the pressure of the pressurized therapeutic agent decreases once the needle opens into the cavity, and the needle will cease to move forward when force is applied to the pushing plunger. At Stage III-b, the therapeutic is injected into the cavity at a pre-selected rate when force is applied to the needle plunger. The force needed to administer the therapeutic can depend on the density and viscosity of the therapeutic, the frictional or sliding force between the plunger and the medicament chamber, the inner diameter of the medicament chamber, the length of the needle, and the inner diameter of the needle. In some embodiments, once the needle reaches the cavity, the system can continue advancing the pushing plunger to inject the therapeutic agent into the cavity without a break. In some embodiments, the pushing plunger can be stopped once the needle lumen reaches the cavity, and then re-started to inject the therapeutic agent into the cavity.

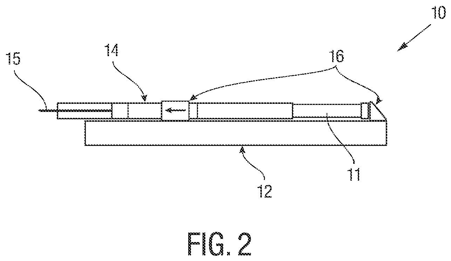

In reference to FIG. 2, the motorized injection system 10 of the present disclosure includes a housing or support platform 12 that supports an injection system 14, a drive assembly or drive mechanism 16 and one or more sensors to measure the load on the injection system or its components. The support platform 12 is configured for anchoring the auto-injection system in a fixed position relative to a site of injection. In some embodiments, the motorized injection system 10 can further include a controller in communication with the drive assembly or the one or more cells to process control the injection. In some embodiments, the controller may be configured to monitor the rate and force of the injection. In some embodiments, the controller may be configured to provide a feedback mechanism to the user. In some embodiments, the controller may be configured to trigger movement of either the syringe, the pushing plunger, or both in response to feedback from the load cell or load cells alone or in combination with tracking of the distances moved by the pushing plunger, the syringe or the differential motion of one relative to the other.

In some embodiments, the injection system may include a syringe comprising a syringe barrel defining a medicament chamber for storing a therapeutic agent, a needle 15 in fluid communication with the medicament chamber to administer the therapeutic agent from the medicament chamber, and a plunger 11 slidably disposed within the syringe barrel and being configured to expel the therapeutic agent from the medicament chamber through the needle 15.

In some embodiments, a standard syringe can be used, such that the medicament chamber can have a volume of between about 0.1 ml and 20 ml, but larger or smaller syringes can also be used. In some embodiments, the medicament chamber can have a volume of approximately 0.1 ml, 0.5 ml, 1 ml, 3 ml, 5 ml, or 10 ml prior to the displacement of the fluid.

In some embodiments, the needle may be a standard needle between 34G and 25G. In some embodiments, the needle may be a standard 30G needle. Various needle sizes can be used to deliver therapeutic treatment to the SCS, as discussed above. In some embodiments, particularly for higher viscosity formulations, for example, greater than 10 centipoise, needles with larger lumens may be used. The pre-insertion step required to block the fluid flow may set a limit to the range of needle lumen diameter and bevel size that can be used effectively to target the SCS. In some embodiments, with a minimal human scleral thickness in mind, optimal results can be obtained by limiting the pre-insertion depth to less than or equal to approximately 0.5 millimeters (for example, between about 0.05 mm to 0.5 mm) if the needle is inserted perpendicular to the scleral surface. If inserted at an angle other than perpendicular, one can sufficiently insert a needle with longer bevel without piercing through the sclera. For example, based on geometrical correlation, a 30-gauge needle with the standard bevel (angle: 12 degrees, length: 1.45 mm) inserted at an angle less than or equal to approximately 20° to the surface will reach less than 0.5 millimeters deep when measured normally from the surface. Similarly, larger needles with longer bevel lengths can also be used. Shorter bevels allow for a greater range in angles of pre-insertion for a given needle size. Broadly speaking, needles with outer diameters smaller than the scleral thickness of approximately 0.5 millimeters are readily usable to access the SCS and the angle of needle insertion is determined based on the beveled tip length. In some embodiments, the volume of the medicament chamber is between 20 and 200 microliters. For improved haptics and signal to noise ratio for the distance tracking elements, in some embodiments, the stroke length of the pushing plunger to deliver the therapeutic fluid or suspension is at least 1 centimeter in length. For some embodiments, the flow rate of injection is targeted to be between 0.2 and 20 microliters per second on average. In some embodiments, the syringe barrel is lined in silicone oil, silicone rubber, rubber, glass, polytetrafluorethylene, or polypropylene to minimize adsorption of the therapeutic to the syringe barrel inner surface.

In some embodiments, the friction between the syringe barrel and the plunger can be designed to optimize the performance of the system. For example, the system can be sized such that the static and kinetic friction coefficient are approximately equal so that there is no unintended acceleration of the needle. On the other hand, the static friction coefficient can be higher than the kinetic friction coefficient so that after the needle stops in the cavity, if it has a high force barrier to overcome. High static friction of the needle plunger also allows for high fluid flow rates during injection while maintaining the needle tip position. In some embodiments, the needle-plunger-kinetic-friction is high enough to arrest the needle motion as soon as the lumen is exposed to the cavity. However, the kinetic coefficient can still be limited such that the internal pressure inside the syringe barrel is not so high as to result in a break-down of or injury to the tissue.

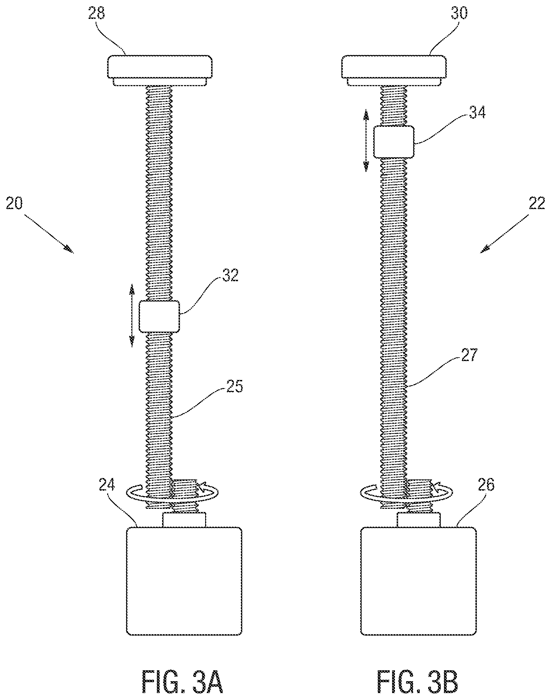

In reference to FIG. 3A and FIG. 3B, in some embodiments, the drive assembly is configured to independently operate a syringe barrel (for example, to pre-insert the needle into the tissue) and a syringe plunger. The drive assembly is designed to translate the syringe barrel relative to the support platform 12, toward the patient to pre-insert the needle into the tissue, and then away from the patient to withdraw the needle from the tissue. The drive assembly also applies the force on the plunger to translate the plunger within the syringe barrel to advance the needle through the tissue toward the cavity and to dispense the therapeutic agent from the medicament chamber. In some embodiments, the drive assembly may comprise separate drivers for the syringe barrel and the plunger. In some embodiments, each such driver may include a linear actuator linked to the syringe barrel or the plunger and a load cell for sensing the load on the syringe barrel or the plunger. As shown in FIGS. 3A-3B, the drive mechanism 20, 22 can include a motor 24, 26, respectively, that is configured to drive the movement of the drive mechanisms 20, 22, such as for example, through a lead screw 25, 27 and an actuator 32, 34. Each of the drive mechanisms 20, 22 can also include at least one load cell 28, 30 that is configured to sense the load on the syringe barrel or the syringe plunger, respectively. In some embodiments, such design may allow the system to advance the syringe barrel without advancing the plunger, and while measuring the force. For example, when the syringe barrel moves, the pushing plunger can move with it. While pre-inserting the needle, the force is measured on the syringe barrel. After pre-insertion, the force is measured on the pushing plunger. During pre-insertion, once the force is sensed, indicating contact of the needle with the tissue, the needle is moved forward a set distance that embeds the lumen of the needle into the tissue.

In some embodiments, the linear actuator may be a mechanical actuator comprising for example, a lead screw and a nut or gear driven by an electric motor, but other designs may be used. In some embodiments, pneumatic, hydraulic, electromechanical, magnetic or other types of linear actuators may also be used. In some embodiments, a single actuator may be used to drive both the syringe barrel and the plunger. Different motion of the syringe barrel and the plunger can be achieved by engaging/disengaging gear mechanism. In some embodiments, linear motion of the pushing plunger can be accomplished by applying hydraulic pressure.

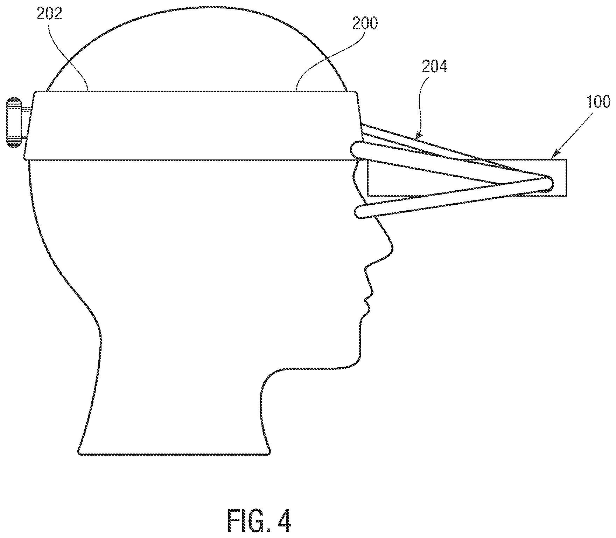

In reference to FIG. 4, the motorized injection system of the present disclosure can also include means for attaching and stabilizing the system relative to the injection site. In this manner, the user can have free hands, while maintaining a fixed x-y coordinate for the injection, in the cases where the syringe barrel is held in place by the attaching and stabilizing system. In other cases, the syringe barrel is fixed only in the x-y- plane, and the syringe can be pushed or pulled toward or away from the eye in the z- plane by the user for the injection. In some embodiments, for the SCS injection the motorized injection system can include an adjustable headband 200 that can be fit to size by ratcheting or other mechanism, such as a ratcheting size adjuster 202 shown in FIG. 4. In some embodiments, other parts of the patient's face could be used to mount the system such as the eye sockets, temple, jaws, ears, nose etc. In some embodiments, the motorized injection system may be attached to a stationary brace and the patient can press his or her face against the brace (similar to an eye exam). Additionally or alternatively, the motorized injection system of the present disclosure can include a guide support 204, such as a tripod, bipod or unipod support, that is either attached to the headband, motorized injection system 10 or both to stabilize the injection system about the eye. In some embodiments, the motorized injection system of the present disclosure can further include a contact pad that can be pressed against the tissue being injected with the therapeutic agent to stabilize the insertion site or adjust the insertion angle. In some embodiments, a stabilizing tripod mentioned above can be used to hold the eye in place. For example, for ocular injections, such pad can be sized and shaped to prevent eye rolling when pressed against the sclera by the user (relative angle of injection controlled). In some embodiments, the component used to prevent significant rotation of the eye may be an independent device that is not attached to the motorized injection system.

### Sensors and Feedback Loops

**In** some embodiments, the motorized system of the present disclosure includes a plurality of sensors that can measure one or more parameters throughout the injection process. The one or more sensors may communicate with the controller to implement one or more feedback loops to control various steps of the injection process. Various types of sensors or other mechanisms can be used to control the injection steps, including but not limited to load cells and sensors, such as pressure sensors, strain sensors, and/or force sensors, as will be discussed in more detail below.

**In** some embodiments, the load that the drive assembly applies onto the syringe barrel and onto the pushing plunger may be measured and communicated to the controller. In some embodiments, the load can be measured using one or more load cells. Such load cells can be embedded in or otherwise configured to receive a signal from the needle, the needle plunger, the pushing plunger or both. In some embodiments, such load may be measured by the torque experienced by the motor based on its correlation to the current drawn by the motor. In some embodiments, the motorized injection system may further include one or more sensors to monitor the position or movement of the injection system as the whole or the syringe barrel or the plunger individually. Such information may be used, for example, to determine the distance travelled by the injection system, the syringe barrel, or the plunger, the needle plunger, in combination or individually. In some embodiments, the motorized system may include one or more sensors to monitor the position or speed at which the syringe barrel or the plunger, in combination or individually, as they move. For example, such information may be used to control the flow rate of the therapeutic agent or to prevent the pushing plunger from overshooting the desired injection volume. In some embodiments, the flowrate may be monitored using a high precision flow sensor including microfluidic mass flow sensors.

In some embodiments, the system of the present disclosure can also measure the pressure in the syringe. In some embodiments, the pressure can be measured indirectly by monitoring the load on the plunger. In some embodiments, the system can be configured such that both the kinetic and static frictions coefficients between the plunger and the syringe barrel are close to 1 to sense the fluid pressure more accurately. In some embodiments, the motorized injection system may further include one or more pressure sensors, force sensors, or strain sensors for a direct measurement of the pressure of the therapeutic agent in the medicament chamber.

In some embodiments, the relative position of the pushing plunger to either the needle plunger in the case of an auto-stop syringe or the needle hub in the case of a standard syringe, is monitored to determine the volume of therapeutic drawn into the syringe or injected into the cavity. In some embodiments the distance of travel measured following pre-insertion can have limits set so as to minimize the chances of overshoot.

In some embodiments, one or more feedback loops may be implemented based on the load distributions during Stages I - III-a (as discussed above in connection with FIGS. 1A-1B). In some embodiments, a feedback loop can monitor the axial load on the system during the pre-insertion of the needle into the tissue at Stage I. In some embodiments, a load cell can be configured to measure, directly or indirectly, the axial load experienced by the needle. During the pre-insertion, the axial load on the needle increases as the needle is pushed into the tissue and drops once the needle punctures the tissue. Accordingly, in some embodiments, a feedback loop is configured to monitor the load on the needle to monitor the pre-insertion and to determine when the needle enters the tissue. In some embodiments, a feedback loop can be configured to control pressurizing the therapeutic agent in the medicament chamber once the needle is embedded into the tissue. In some embodiments, the load on the plunger and/or the distance travelled by the plunger can be monitored to determine when the therapeutic agent is pressurized to a desired pressure. In some embodiments, a feedback loop is configured to monitor the movement of the needle through the tissue toward the cavity during Stage II and into Stage III-a. In some embodiments, the pressure of the therapeutic agent in the syringe barrel can be monitored by, for example, measuring the load on the pushing plunger. In some embodiments, the applied load can be sinusoidal at high frequency and the response curve can be measured from the sensor to measure the internal pressure. The needle can be advanced through the tissue until the load on the plunger or the pressure of the therapeutic agent decreases, indicating that the needle has reached the cavity, so that the lumen of the needle is in fluid communication with the cavity and the therapeutic agent can be delivered into the cavity. In some embodiments, a feedback loop can be provided to monitor the injection of the therapeutic agent into the cavity at Stage III-b. In some embodiments, such feedback loop may monitor the flow rate of the therapeutic agent by, for example, monitoring the speed of travel of the plunger or the pressure of the therapeutic agent. In some embodiments, the feedback loop may monitor the distance traveled by the plunger (correlating to a desired injection volume). In some embodiments, the load on the plunger can be monitored as the plunger advances through the syringe barrel. When the plunger reaches the end of the barrel or some other stop that prevents any further distal movement of the plunger, the load on the plunger would start to increase as the drive mechanism continues to apply a force on the plunger in the distal direction. Such increase in the load of the plunger at the end of Stage III-b would indicate that the injection has been completed and the needle can be withdrawn from the patient.

Various embodiments of the present disclosure may include one or more feedback loops discussed above depending on the design of the system or the level of control desired by the user, among other considerations.

### Syringe Design

**In** some embodiments, a regular syringe may be used in the motorized injection system of the present disclosure. Such syringe can include a syringe barrel for holding one or more injection agents, a needle attached to the syringe barrel in fluid communication with the barrel and a plunger to expel injection agent from the syringe barrel through the needle.

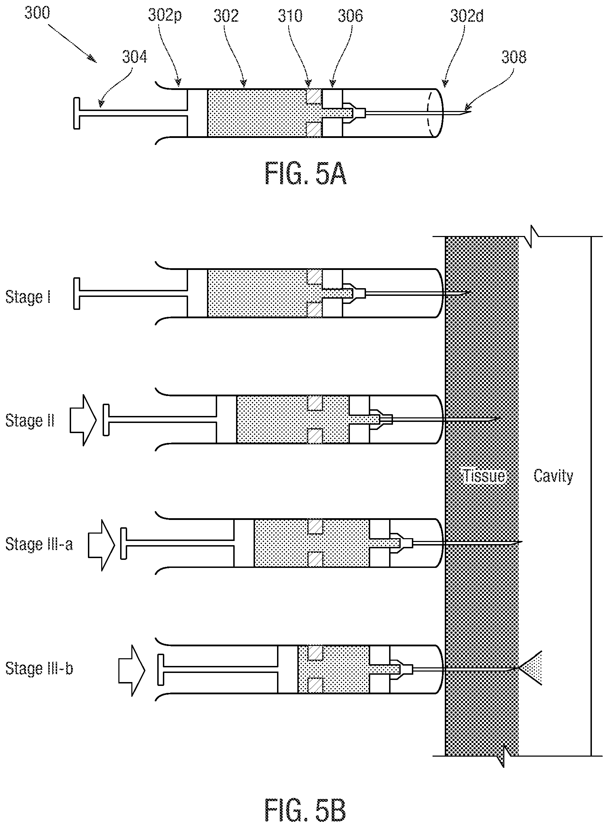

**In** some embodiments, an adjustable injection system (also referred to as an auto-stop injection system) that automatically self-adjusts the depth the needle penetrates into the tissue/cavity can be used. In reference to FIG. 5A, an auto-stop syringe 300 may include a syringe barrel 302 having a proximal end 302p and a distal end 302d, a pushing plunger 304 moveably disposed in the syringe barrel 302 and forming a seal with the syringe barrel, a needle plunger 306 moveably disposed in the syringe barrel distally to the pushing plunger such that a medicament chamber is defined in the syringe barrel between the pushing plunger and the needle plunger. In some embodiments, a needle plunger seat 310 can be provided to control the movement of the needle plunger in the proximal dirrection, and in some embodiments, the pushing plunger may be configured to be advanced past the needle plunger seat.

A movable needle 308 is supported by the needle plunger so that the movement of the needle plunger may also move the needle, the needle being in fluid communication with the medicament chamber to deliver a therapeutic agent from the medicament chamber to the patient. The needle can be connected to the needle plunger using multiple techniques. In some embodiments, the needle is inserted into the rubber plunger and secured with waterproof adhesive. In some embodiments, the plunger could be molded around the needle. In some embodiments, a needle with threads on the outer surface could be screwed into the plunger.

FIG. 5B further illustrates the operation of the auto-stop syringe (the drive mechanism/support assembly not shown). At Stage I, the needle is pre-inserted into the tissue (for example, sclera of the eye). In some embodiments, the needle can be inserted tangentially to the sclera with the needle tip pointing to the posterior segment of the eye. Next, at Stage II, a force is applied to the pushing plunger, which pushes the needle plunger forward to advance the needle deeper through the tissue toward a cavity (for example, the suprachoroidal space of the eye). At Stage III-a, the needle tip enters the cavity, and once the lumen of the needle opens up into the cavity, the needle plunger automatically stops thus limiting the depth the needle penetrates into the cavity. The precision and miniaturization of the auto-stop syringe allows the needle plunger to precisely target and stop at a thin potential cavity, such as the suprachoroidal space. At Stage III-b, as the operator continues to push on the pushing plunger, the therapeutic agent in the medicament chamber is delivered into the cavity, while the needle holds its position at the tissuecavity interface. In some embodiments, the vector of fluid flow is parallel to the suprachoroidal space to provide broad coverage of the posterior segment of the eye instead of the fluid force being used to displace the choroidal and retinal tissues radially.

An exemplary auto-stop syringe for use to deliver therapeutic agents into the suprachoroidal space is disclosed in US Application 16/469,567, filed June 13, 2019 and PCT Application PCT/US2020/051702, filed on September 20, 2020. In some embodiments, such design variables as the syringe geometry, needle geometry, flow rate, viscosity, and frictional forces are related and can be designed as discussed in Chitnis, G.D., Verma, M.K.S., Lamazouade, J. et al. A resistance-sensing mechanical injector for the precise delivery of liquids to target tissue. Nat Biomed Eng 3, 621-631 (2019). In some embodiments, the insertion force can be taken into account to select various design variables. As a result, the system is capable of delivering drug and gene therapies that benefit from localization to the SCS including those that treat diseases and disorders of the choroid and the retina. It should be noted that while the present disclosure describes the instant injection system in connection with drug delivery to the SCS cavity, the presently disclosed systems and methods can be used to deliver therapeutic agents to other voids or cavities of the human body.

In some embodiments, the auto-stop syringe is pre-filled with a therapeutic. In some embodiments, the therapeutic is contained in one or more vials, which interface via a rapid fill port to the syringe barrel (for example, as described in co-pending PCT Application PCT/US2020/051702, filed on September 20, 2020.

### Operation of the Motorized Injection System

Either prior to filling with a therapeutic or after filling with a therapeutic, depending on whether the filling process is automated, the motorized injection system can be connected to the head and/or eye of the patient. In some embodiments, an adjustable headband can be secured around a patient's head. In some embodiments, the distal end of the motorized injection system can be anchored to external landmarks around the ocular orbit.

The position of the motorized injection system can be adjusted to achieve a desired angle of needle insertion, which would depend on the bevel, to embed the lumen into the tissue. Once the motorized injection system is in place, the tip of the needle can be located near the surface of the sclera, for example, within about 2 centimeters, within about 1 centimeter, or within about 0.5 centimeters from the surface of the sclera. In some embodiments, such distance can be between about 0.1 and about 2 cm, about 0.1 and about 1 cm, or about 0.1 and about 0.5 cm. In some embodiments, an acoustic or laser range finder may be used to assist with the initial positioning of the needle tip. Next, the user provides a signal via a button or touch screen to initiate the injection process.

### Motorized syringe drivers for auto-stop syringes

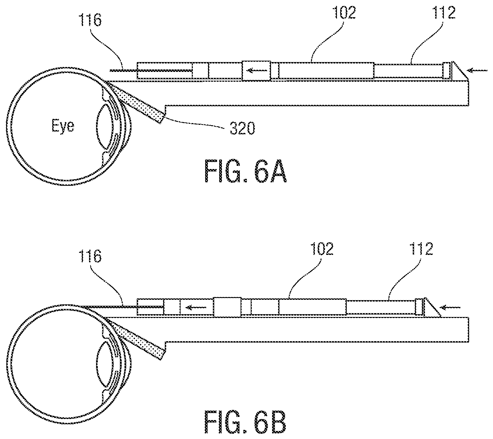

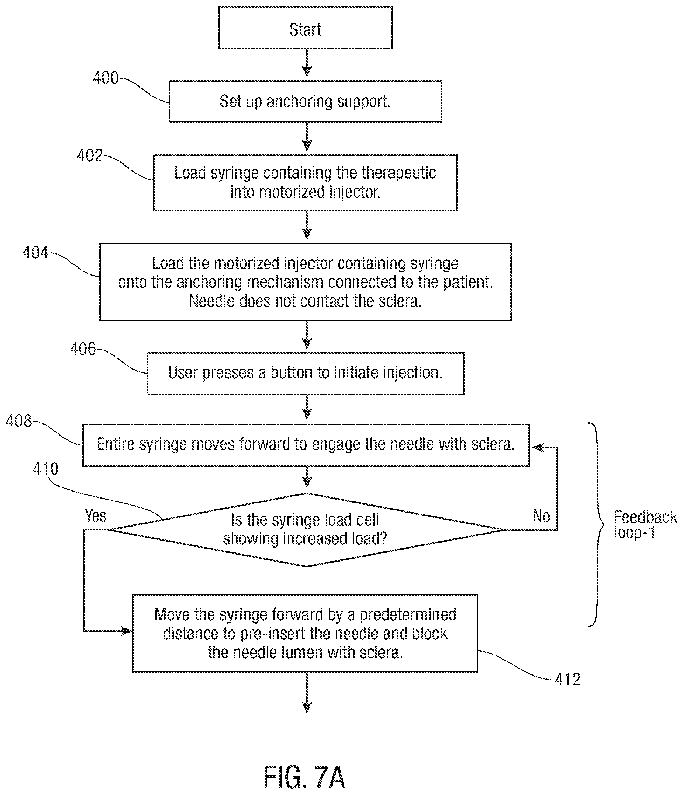

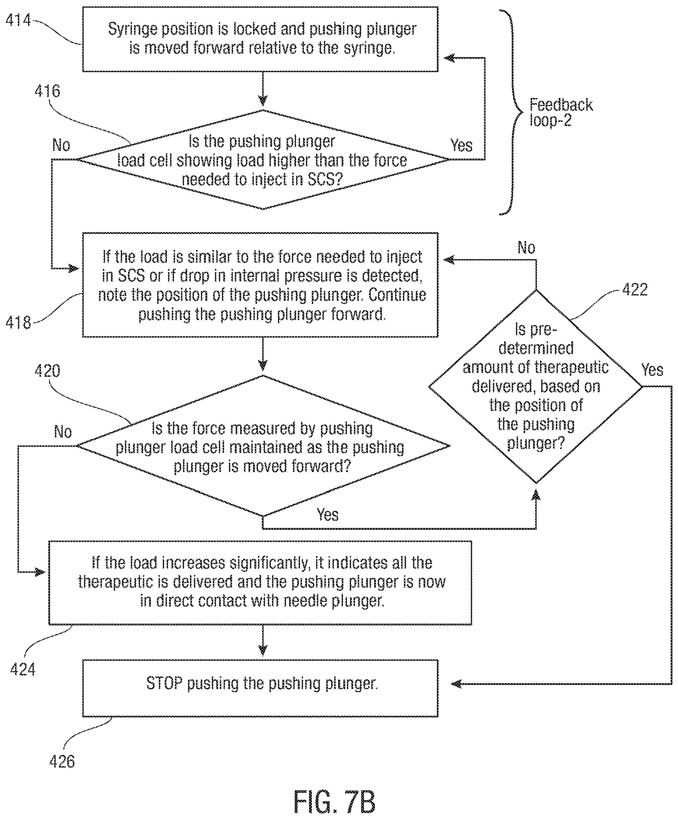

By way of a non-limiting example, the use of the motorized injection system with an auto-stop syringe is described in reference to FIGS. 6A-6D and FIGS. 7A-7B.

In reference to FIG. 6A, after the user initiates the injection process, the auto-stop syringe is advanced towards the surface of the eye. In some embodiments, to achieve this, the syringe barrel 102 and the pushing plunger 112 are moved in unison at the same velocity towards the eye until a load cell detects the needle 116 embedding in the sclera. During the advancement of the auto-stop syringe, the load on the syringe barrel, the needle guide, or the needle is sensed, for example, by a load cell or force sensor. In some embodiments, this advancement can be performed manually. Once the needle tip reaches the sclera, the load will increase with contact force. In some embodiments, the load measured at this stage is the axial load experienced by the needle. The load continues to increase until the needle pierces the sclera, at which point the load drops. Once the load drops, signaling the piercing of the sclera, the auto-stop syringe and floating needle are advanced until the lumen of the needle is fully imbedded in the sclera. In some embodiments, to determine when the lumen of the needle is fully embedded, the system can rely on a fixed advancement of the needle after detecting the scleral puncture. In some embodiments, the complete insertion can be determined, by advancing the pushing plunger slightly to see if it starts building pressure or leads to leakage.

Once the needle is imbedded into the sclera, the movement of the syringe barrel is stopped, and the pushing plunger is advanced in the syringe barrel to pressurize the contents of the syringe barrel. In some embodiments, the pushing plunger is advanced a pre-specified or user-specified distance or until a pre-specified or user-specified load is reached on the load cell attached to the pushing plunger or its fixture confirming that the lumen of the needle is fully imbedded in the sclera. In some embodiments, this step is skipped.

In some embodiments, an optional elastomer contact pad 320 can be used to prevent eye rolling pressed against sclera by an operator of the device, such as a physician. This can allow the relative angle of injection to be controlled.

In reference to FIG. 6B, with the needle pre-inserted into the sclera, the syringe barrel 102 is locked in place, and the pushing plunger 112 is advanced forward to advance the needle tip through the sclera. Pressure within the syringe barrel is maintained while the needle advances through the sclera due to the low water permeability of the sclera causing the fluid to be trapped inside the auto-stop syringe. The therapeutic agent cannot be dispensed into the sclera due to the backpressure produced by the dense tissue of the sclera. Instead, advancing the pushing plunger also advances the needle plunger and the needle toward the SCS. The syringe barrel holder stops and the plunger continues to move thereby forcing the second plunger (i.e. the needle plunger) towards the SCS.

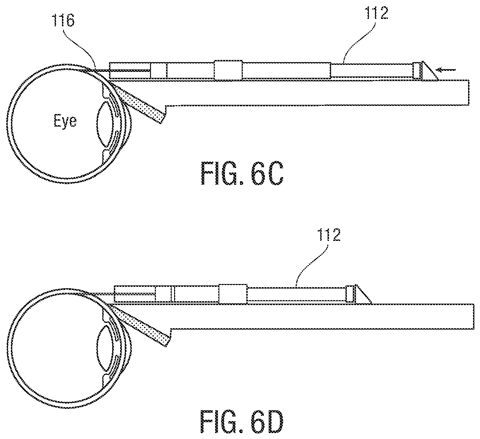

In reference to FIG. 6C, when the needle 116 reaches the SCS, the fluid pressure within the syringe barrel drops, which can be sensed by the pushing plunger load cell or a pressure sensor. In some embodiments, detection of relative motion of the pushing plunger moving closer to the needle plunger can also or alternatively be used to identify when cavity is reached. Due to the decrease in the backpressure produced by the SCS in comparison to the backpressure produced by the sclera, the needle stops advancing and, instead, the therapeutic agent is expelled through the needle into the SCS. The pushing plunger 112 continues to be advanced at a pre-determined or user-determined velocity and/or a predetermined distance or user-determined distance to inject the therapeutic, in the form of a fixed volume of liquid, into the SCS.

In reference to FIG. 6D, once the pushing plunger 112 has moved a pre-specified or user-specified distance, corresponding with a desired injection volume, or the pushing plunger senses an increased load corresponding to having reached the needle plunger, the pushing plunger fixture ceases advancing. In some embodiments, such increased load can be set depending on the static friction of the needle plunger, so that the pushing plunger cannot advance the needle plunger.

After the therapeutic agent has been delivered to the SCS, the user can remove the syringe needle from the eye. In some embodiments, the syringe can be removed manually. In some embodiments, the entire auto-stop syringe is retracted away from the eye until the needle is no longer contacting the sclera. This can be achieved by returning the auto-stop syringe to the starting positions, or by returning at least to the point at which the needle tip had initially sensed an increase in load, corresponding with contacting the scleral surface.

In some embodiments, one or more feedback loops can be used to monitor the operations of an auto-stop syringe. In some embodiments, a first feedback loop can monitor the pre-insertion of the needle into the sclera. For example, during insertion of needle into the sclera (movement of the complete syringe towards the eye), a load cell attached to the needle or syringe barrel can register increasing force until the needle punctures the sclera. On puncture, there is a drop in load and the needle is further then advanced a pre-determined distance until the lumen of the needle is imbedded into the sclera and then discontinues advancing the complete syringe.

In some embodiments, a second feedback loop can be provided to monitor the advancement of the needle through the sclera. For example, once the needle pre-insertion is complete, the syringe barrel is locked in place and the pushing plunger advances while the load on the pushing plunger is measured. The load increases on the pushing plunger and may plateau as the needle advances through the sclera. The load falls once the needle lumen reaches the SCS.

In some embodiments, a third feedback loop may be provided to monitor the injection of the therapeutic agent into the SCS. For example, the pushing plunger can be advanced at a pre-specified velocity until a specific distance (correlating to a desired injection volume) is reached, or until the pushing plunger reaches the needle plunger to avoid needle overshoot.

As described in more detail in FIGS. 7A-7B, a method for delivering a therapeutic into the eye can begin with setting up an anchoring device in step 400. In step 402, a syringe can be loaded containing the therapeutic into a motorized injector. In step 404, the motorized injector containing syringe can be loaded onto the anchoring mechanism connected to the patient (needle does not contact the sclera). In step 406, a user can press a button or other mechanism to initiate the injection. In step 408, the entire syringe can move forward to engage the needle with the sclera. In step 410, if the syringe load cell shows an increase in load, the syringe is moved forward by a predetermined distance to pre-insert the needle and block the needle lumen with sclera (step 412). If not, then the syringe continues to move forward (step 408), until the load increases.

Once the syringe barrel load cell shows an increase in load, the syringe barrel position is locked and pushing plunger is moved forward relative to the syringe in step 414. In step 416, if the pushing plunger load cell is showing load higher than the force needed to inject in SCS, then the pushing plunger is moved forward relative to the syringe (step 414). If the pushing plunger load cell is not showing load higher than the force to inject in SCS, then in step 418 the pushing plunger can continue to be pushed forward. If the load is similar to the force needed to inject in SCS or if a drop in internal pressure is detected, note the position of the pushing plunger so the distance moved by the pushing plungercan be monitored, which can be used, for example,- to prevent the pushing plunger from bumping into the needle plunger or to monitor the amount of the injection agent delivered to SCS.

In step 420, if the force measured by the pushing plunger load cell is maintained as the pushing plunger moves forward, then it is determined whether or not the pre-determined amount of therapeutic has been delivered based on the position of the pushing plunger (step 422). If it has not, then the system continues to push the pushing plunger forward (step 418). If it has, then the system stops pushing the pushing plunger forward (step 426). If the force measured by the pushing plunger load cell is not maintained as the pushing plunger is moved forward (step 420), then in step 424, if the load increases significantly, it indicates all the therapeutic is delivered and the pushing plunger is at the end of the injection chamber. The system then stops moving the pushing plunger forward (step 426).

### Motorized syringe drivers for standard syringes

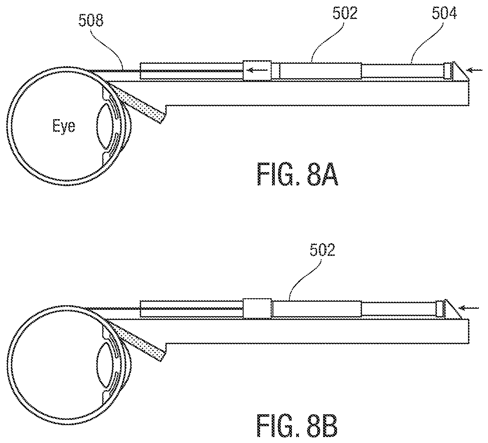

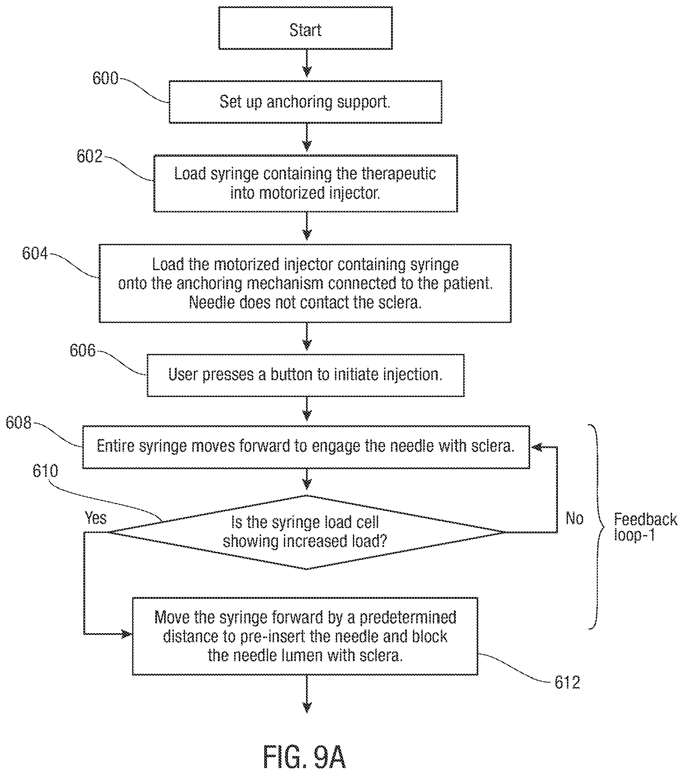

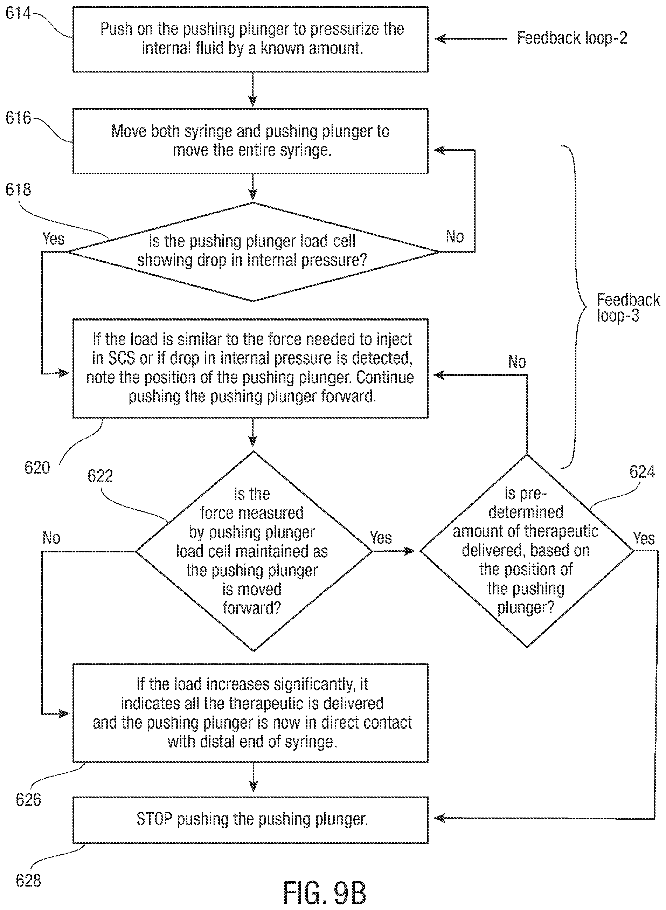

By way of a non-limiting example, the use of the motorized injection system with an auto-stop syringe is described in reference to FIGS. 8A-8E and FIGS. 9A-9B.

In reference to FIG. 8A, after the user initiates the injection process, the entire syringe advances towards the surface of the eye by moving the barrel 502 of the syringe and the pushing plunger 504 at approximately the same velocity until a load cell detects the needle embedding in the sclera. During the advancement of the syringe, the load cell that is contacting the barrel of the syringe, or the needle 508, is sensing load. Once the needle tip reaches the sclera, load will increase with contact force. Load continues to increase until the needle pierces the sclera, at which point the load drops. Once the load drops, signaling the piercing of the sclera, the syringe and needle are advanced until the lumen of the needle is fully imbedded in the sclera.

In reference to FIG. 8B, once the needle is imbedded into the sclera, the syringe barrel 502 is held in place and remains stationary, while the syringe plunger is advanced a pre-specified or user-specified distance or until a pre-specified or user-specified load is reached on the load cell attached to the pushing plunger or its fixture. This movement of the plunger increases the pressure of the therapeutic agent, which is registered as load on the syringe plunger.

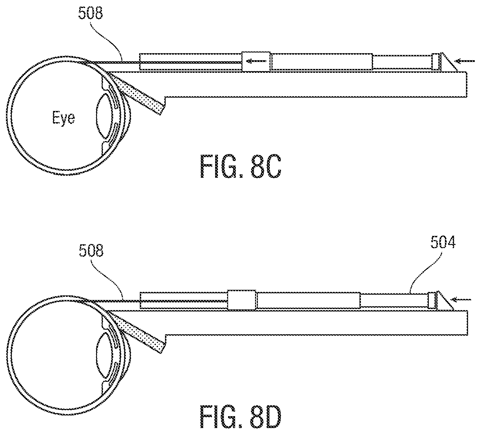

In reference to FIG. 8C, next, the full syringe is advanced into the sclera by the motorized syringe driver causing a tip of the needle 508 to advance through the sclera. Pressure within the syringe barrel is maintained while the needle as fluid egress is blocked, and the syringe advances through the sclera due to the low water permeability of the sclera causing the fluid to remain inside the syringe, until the lumen of the needle reaches the SCS.

In reference to FIG. 8D, once the lumen of the needle 508 reaches the SCS, the fluid pressure (i.e. the load) drops on the pushing plunger since the lumen is no longer blocked and fluid can egress, which is recorded by the load monitoring the syringe plunger. On sensing the drop in the load on the syringe plunger, the syringe barrel stops in place. The pushing plunger is then advanced at a pre-determined or user-determined velocity to inject the therapeutic into the SCS.

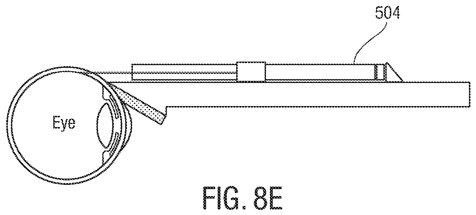

In reference to FIG. 8E, once the pushing plunger 504 has moved a pre-specified or user-specified distance, corresponding with a desired injection volume or the entire payload, or the pushing plunger senses an increased load corresponding to having reached the end of the syringe barrel, the pushing plunger fixture ceases advancing. Delivery of entire payload can be detected using load-cell based on increased force as the plunger engages with the distal end of the syringe when the syringe is empty.

After the therapeutic agent has been delivered to the SCS, the user can remove the syringe needle from the eye. In some embodiments, the syringe can be removed manually. In some embodiments, the entire syringe is retracted away from the eye until the needle is no longer contacting the sclera. This can be achieved by returning the syringe to the starting positions, or by returning at least to the point at which the needle tip had initially sensed an increase in load, corresponding with contacting the scleral surface.

In some embodiments, one or more feedback loops can be used to monitor the operations of an auto-stop syringe. In some embodiments, a first feedback loop can be provided to monitor the insertion of the needle into the sclera. For example, when the needle is pre-inserted into the sclera (movement of the complete syringe towards the eye), a load cell attached to the needle or syringe barrel registers increasing force until the needle punctures the sclera, but then there is a drop in load. After the drop is detected, the needle can then be advanced a pre-determined distance until the lumen of the needle is imbedded into the sclera and the syringe barrel can be stopped. In some embodiments, a second feedback loop can be provided to pressurize the contents of the syringe barrel. For example, once the pre-insertion is complete, the syringe barrel is locked in place and the pushing plunger advances while the load on the plunger or the pressure of the therapeutic agent is measured until a pre-specified load, distance or pressure is reached to pressurize the therapeutic agent. In some embodiments, a third feedback loop is provided to monitor the load on the needle as it is being advanced through the sclera. In some embodiments, the load on the needle can be monitored indirectly. For example, once the fluid contents of the syringe are pressurized, the entire syringe is advanced until the load drops on the pushing plunger, indicating that the lumen of the syringe has reached the SCS. In some embodiments, when the needle is fixed to the hub of the syringe, the load on the syringe can be monitored since such load is indicative of the load on the needle. In some embodiments, once the cavity is reached, a fourth feedback loop can be used to deliver the therapeutic agent to the SCS. For example, once the needle enters the SCS, the plunger is then advanced at a pre-specified velocity until a specific distance (correlating to a desired injection volume) is reached, or until the pushing plunger reaches the needle plunger to avoid needle overshoot.

As described in more detail in FIGS. 9A-9B, a method for delivering a therapeutic into the eye can begin with setting up an anchoring device in step 600. In step 602, a syringe can be loaded containing the therapeutic into a motorized injector. In step 604, the motorized injector containing syringe can be loaded onto the anchoring mechanism connected to the patient (needle does not contact the sclera). In step 606, a user can press a button or other mechanism to initiate the injection. In step 608, the entire syringe can move forward to engage the needle with the sclera. In step 610, if the syringe load cell shows an increase in load, the syringe is moved forward by a predetermined distance to pre-insert the needle and block the needle lumen with sclera (step 612). If not, then the syringe continues to move forward (step 608), until the load increases.

In step 614, the pushing plunger is pushed to pressurize the internal fluid by a known amount, and in step 616, both the syringe and the pushing plunger are moved to move the entire syringe. In step 618, it is determined whether the pushing plunger load cell is showing a drop in internal pressure. If it has, and if the load is similar to the force needed to inject in SCS or if a drop in internal pressure is detected, note the position of the pushing plunger and continue pushing the pushing plunger forward (step 620). If it has not, move both the syringe and the pushing plunger to move the entire syringe (step 616).

In step 622, if the force measured by the pushing plunger load cell is maintained as the pushing plunger moves forward, then it is determined whether or not the pre-determined amount of therapeutic has been delivered based on the position of the pushing plunger (step 624). If it has not, then the system continues to push the pushing plunger forward (step 626). If it has, then the system stops pushing the pushing plunger forward (step 628). If the force measured by the pushing plunger load cell is not maintained as the pushing plunger is moved forward (step 622), then in step 626, if the load increases significantly, it indicates all the therapeutic is delivered and the pushing plunger is now in direct contact with needle plunger. The system then stops moving the plunger forward (step 628).

In particular, in some embodiments, In step 614, the plunger is pushed to pressurize the internal fluid by a known amount, and in step 616, both the barrel and the plunger are moved to move the entire syringe. In step 618, internal fluid pressure gets checked continuously. If the load cell shows drop in pressure, note the position, hold the position of the barrel and continue pushing forward on the plunger to deliver the therapeutic in the SCS space. In step 618, if the plunger load cell has not yet registered a drop in load, then the entire syringe is advanced. When the plunger load cell does register a drop in load, the syringe barrel ceases advancing, and only the pushing plunger is advanced, in step 620. The pushing plunger load cell continues to be monitored, step 622. While load on the pushing plunger is monitored, the pushing plunger advances, delivering the therapeutic until the desired volume of the therapeutic has been delivered, step 624, stopping the advancement of the pushing plunger, step 628. Alternatively, the pushing plunger load cell registers an increase in load indicating that the pushing plunger has reached the distal portion of the syringe, step 626, stopping the advancement of the pushing plunger, step 628.

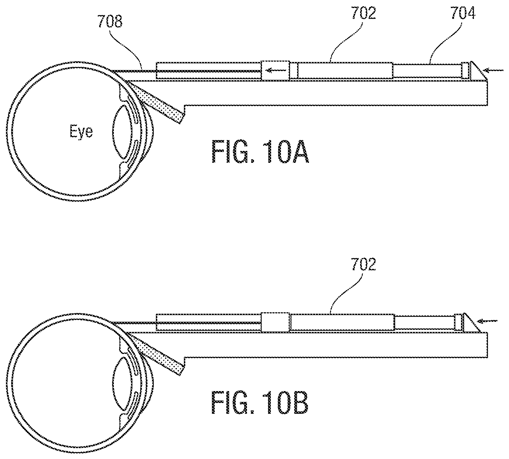

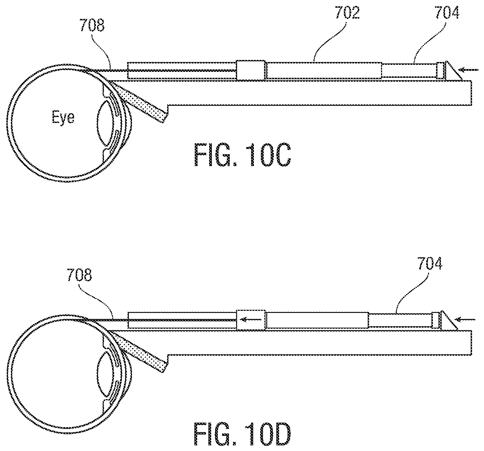

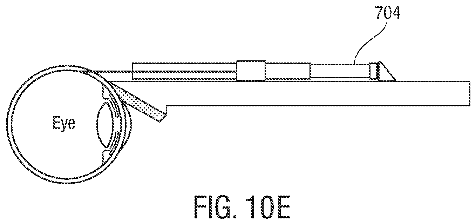

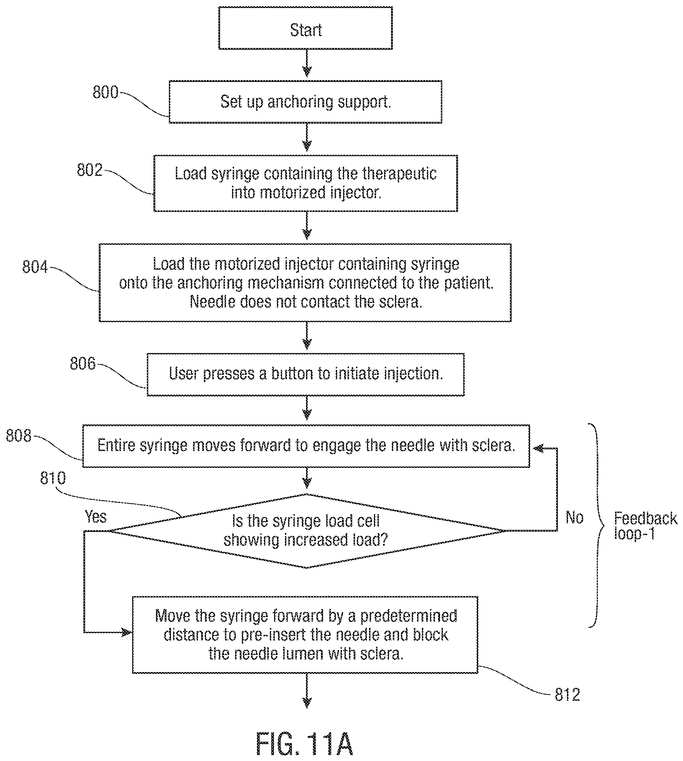

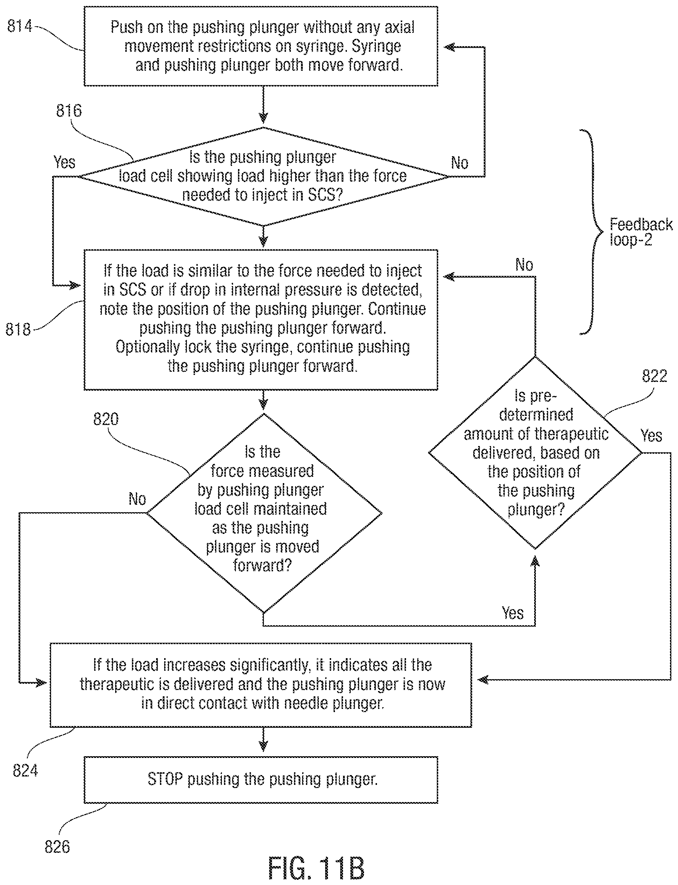

In some embodiments, in reference to FIGS. 10A-10E and FIGS. 11A-11B, after the lumen of the needle is imbedded in the sclera, instead of advancing the full syringe, the fixture attached to the barrel of the syringe is allowed to move freely, and only the pushing plunger fixture is advanced. As shown in FIG. 10A, the syringe barrel 702 and plunger 704 can be moved in unison towards the eye until load cell detects the needle 708 embedding in sclera. In FIG. 10B, with the needle tip embedded, the syringe barrel 702 is held in place and the pushing plunger is advanced to create fluid pressure that registers as load on the pushing plunger. In FIG. 10C, once the pre-pressurized load is reached on the pushing plunger 704, the stop on the syringe barrel is released so that it is free to move, and then the pushing plunger is advanced. In FIG. 10D, when the lumen of the needle 708 reaches SCS, the load will drop on the pushing plunger 704 since the lumen is no longer blocked and fluid can egress; then the pushing plunger is advanced to dispense the therapeutic into the SCS. In FIG. 10E, the pushing plunger 704 can be advanced a set distance to deliver a known volume or can be pushed deliver the entire payload. Delivery of the entire payload can be detected using load-cell based on increased force as the plunger engages with the distal end of the syringe when syringe is empty.

In this way, when the lumen of the needle reaches the SCS, as denoted by a drop in load on the pushing plunger load cell, the syringe barrel can then be locked in place, and the syringe plunger can be advanced, directly injecting the therapeutic into the SCS, essentially creating an auto-stopping needle from a standard syringe, when used in combination with the syringe driver. In such embodiments, a feedback loop may be provided to monitor the movement of the needle through the sclera. In some embodiments, the syringe barrel is not locked in place on reaching the SCS, and the needle remains in the cavity as the pushing plunger is advanced due to the drop in fluid resistance at the lumen of the needle. For example, after the syringe barrel contents are pressurized, the mechanical stop on the syringe barrel is released. Then the plunger is advanced, which advances the needle tip through the sclera until it reaches the SCS. Once there, the needle will automatically stop, as the pressure inside the syringe barrel is reduced by the egress of fluid from the needle tip into the SCS.

As described in more detail in FIGS. 11A-11B, a method for delivering a therapeutic into the eye can begin with setting up an anchoring device in step 800. In step 802, a syringe can be loaded containing the therapeutic into a motorized injector. In step 804, the motorized injector containing the syringe can be loaded onto the anchoring mechanism connected to the patient (needle does not contact the sclera). In step 806, a user can press a button or other mechanism to initiate the injection. In step 808, the entire syringe can move forward to engage the needle with the sclera. In step 810, if the syringe load cell shows an increase in load, the syringe is moved forward by a predetermined distance to pre-insert the needle and block the needle lumen with sclera (step 812). If not, then the syringe continues to move forward (step 808), until the load increases.

Once the syringe load cell shows an increase in load, step 814 includes pushing on the pushing plunger without any axial movement restrictions on syringe, and both the syringe and the pushing plunger move forward. In step 816, if the pushing plunger load is similar to the force needed to inject in SCS or if a drop in internal pressure is detected, note the position of the pushing plunger and continue pushing the pushing plunger forward. Optionally, the system may continue pushing the pushing plunger forward, while the syringe may be locked (step 818). If the pushing plunger load cell is not showing a drop in internal pressure, then the pushing plunger is pushed on as indicated in step 814.

In step 820, if the force measured by the pushing plunger load cell is maintained as the pushing plunger moves forward, then then it is determined whether or not the pre-determined amount of therapeutic has been delivered based on the position of the pushing plunger (step 822). If it has not, then the system continues to push the pushing plunger forward while the syringe position is locked (step 818). If it has, then the system stops pushing the pushing plunger forward (step 826). If the force measured by the pushing plunger load cell is not maintained as the pushing plunger is moved forward (step 820), then in step 824, if the load increases significantly, it indicates all the therapeutic is delivered and the pushing plunger is now in direct contact with the distal end of the syringe. The system then stops moving the pushing plunger forward (step 826).

### Graphical User Interface

A graphical user interface is included in some embodiments. For example, such user interface may allow the user to start the injection, monitor the injection through Stages I-III-b, and to abort the injection as necessary. In some embodiments, there is also a means of providing auditory feedback to the user. In some embodiments, lights, a graphical display and/or sounds are used as an indicator to denote one or more of the following events: setting the angle of insertion, filling the syringe with therapeutic, priming the syringe to get rid of any trapped air, turning on the device, advancing the needle towards the sclera, when the sclera is punctured, when the SCS is reached, when the therapeutic has been delivered, when the needle has been withdrawn from the eye.

In some embodiments, the GUI allows the user to input certain patient parameters including Intra-ocular pressure, scleral thickness, eye size etc. In some other embodiments, the GUI asks for patient information and generates a report after completing injection, and in further embodiments the patient information is obtained via one- or two- dimensional barcode scanner or near field scanner (NFC- Near Field Communication). In some embodiments, the injector can connect to an external server to upload this information and/or download relevant information for the case such as, disease being treated, prescribed therapeutic, and dosage information.

In some embodiments, a display on the motorized syringe driver is used to display instructions to the user and request input from the user as to when to advance to the next step of the injection process beginning with filling the syringe and ending with completion of the SCS injection. In some embodiments, the GUI also allows the user to input injection process parameters, such as, for example, the distance traveled by the system or its component, thresholds for pressure or load on the system or its components, volume of therapeutic to be loaded, volume of therapeutic to be delivered, flow rate of injection, duration of injection, angle of injection, or maximum travel distance of the needle following scleral puncture. In some embodiments, the user can select the desired volume and/or rate of injection. In some embodiments, the position of the pushing plunger, the needle tip and/or the floating plunger are displayed, and/or the load sensed by the needle tip load cell and/or the pushing plunger load cell.

In some embodiments, there is a camera focused on the surface of the tissue that provides a real-time magnified video picture on the display to the user so that they can witness the piercing of the sclera and ultimate withdrawal of the needle. In some embodiments, the camera can assist with the pre-insertion, where the user manually preinserts the needle tip and then activates automated system to complete delivery of the therapeutic agent to the cavity. In some embodiments, the injector may include a scanner for one or two dimensional bar codes to log the disposable syringe and therapeutic used to inject.

### Needle Stopping Distance and Overshoot

As discussed above, in some embodiments, the motorized injection system of the present disclosure can be equipped with one or more safety features to limit or control the needle over-shoot. In some embodiments, additionally or alternatively, the needle overshoot can be controlled by controlling the stopping distance of the needle plunger. The stopping distance is the distance the needle plunger travels after the needle-lumen reaches the cavity and begins the delivery of the therapeutic agent. The stopping distance is determined by how quickly the pressure in the syringe barrel drops below the frictional resistance of the needle plunger. Such distance can be characterized as a relationship (analogous to flow through a hollow needle is characterized by the Hagen-Poiseuille equation) between the frictional resistance of the needle plunger, needle inner diameter, needle length, needle bevel, syringe inner barrel diameter, formulation viscosity, force applied to the pushing plunger, mechanical properties of the device components. In some embodiments, the stopping distance may be predicted as a function of the time needed to drop the pressure and speed at which the needle was traveling. The stopping distance can be dependent on the volumetric elasticity of the syringe assembly and compressibility of the fluid. In some embodiments, the overshoot due to the stopping distance can be controlled by using one or more safety features described above. In some embodiments, the stopping distance can depend on the time required to implement and actuate the motorized feedback loop. As long as some portion of needle opening overlaps with the SCS, the payload will be delivered to the SCS. Hence acceptable stopping-distance is directly related to lumen size and bevel. For example, a 30G needle with 0.160 mm lumen diameter, with a standard 12° bevel angle can overshoot by ~0.8 mm while maintaining lumen-contact with SCS. For optimal fluid flow and maximizing overlap between the lumen and the SCS, the needle should be positioned such that the SCS is placed centrally to the lumen geometry, i.e., for the 30G needle with 12° bevel angle, ~0.4 mm stopping distance would position the SCS centrally to lumen. A stopping distance less than 0.4 mm is also acceptable.

In some embodiments, the stopping distance overshoot is corrected by having the motorized syringe driver retract the barrel of the syringe to ensure SCS is placed centrally to the lumen geometry. For example, for a 30G needle with 12° bevel angle, if the stopping distance is more than 0.4 mm, the needle may be retracted back to place it centrally. In some embodiments, the barrel of the syringe is retracted a fixed distance to counter the volumetric elasticity of the syringe assembly and compressibility of the fluid. In other embodiments, the retraction distance incorporates the position of the syringe barrel when the load drop on the pushing plunger is first detected after traversing the sclera. In some embodiments, the retraction distance utilizes the measured deformation of the sclera in response to contact by the needle prior to puncture. In some embodiments, the retraction distance utilizes the known or measured time required to detect and implement the syringe stopping motorized feedback loop.

### Automated Syringe Filling

In some embodiments, the auto-stop syringe of the present disclosure may be prefilled with the therapeutic agent during manufacturing, as described above. In some embodiments, the auto-stop syringe of the present disclosure may be filled with the therapeutic agent in the doctor's office, compounding pharmacy or surgical suite prior to the administration of the therapeutic agent to the patient. In some embodiments, the therapeutic agent may be provided in a vial for storage and may be transferred to the SCS system by the user only when the therapeutic agent is ready to be administered to the patient.

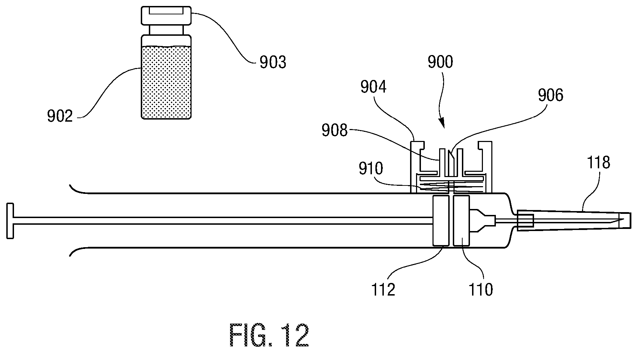

In some embodiments, as shown in FIG. 12, the injection system of the present disclosure is provided with a rapid fill port 900 to enable the loading of the injection agent into the injection chamber from a vial 902. In some embodiments, the rapid fill port 900 includes a receptacle 904 configured to accept the vial 902 to fluidly connect to the vial to the injection chamber. In some embodiments, a hole or passageway is created (e.g., through molding, machining, etc.) through the wall of the syringe barrel proximal to the needle plunger 110, and the receptacle 904 is placed over such hole or passageway.

In some embodiments, when the needle plunger is set in its initial position and the pushing plunger is brought in contact with the needle plunger, the rapid fill port is fluidly connected to the syringe barrel at the site between the sealing elements. Connected to the passageway, partially or fully disposed within it, is a side port fill needle 906 (preferably larger than the injection puncture element, such as an 18 gauge puncture element). Such fill needle can be beveled to pierce the elastomer cap 903 of the therapeutic containing vial 902. In some embodiments, the fill puncture element of the rapid fill port can have its opening on the side of the fill puncture element rather than at the tip. This side port can be covered by a casing or self-sealing puncture membrane 908 that blocks fluid flow when in the closed position. The casing 908 can be disposed within the receptacle and can be biased by a spring 910 to close the port of the fill needle when the vial is not present in its receptacle. In some embodiments, the safety cap 118 may be configured to provide an airtight seal when attached to the injection system.

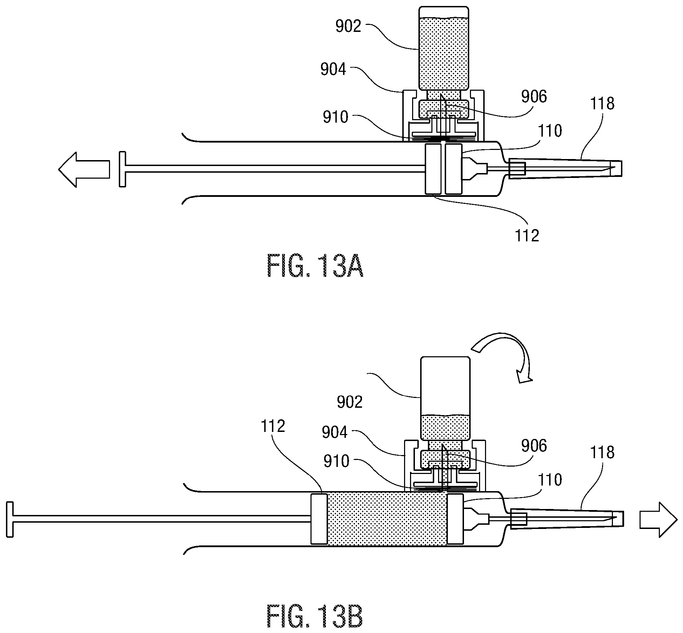

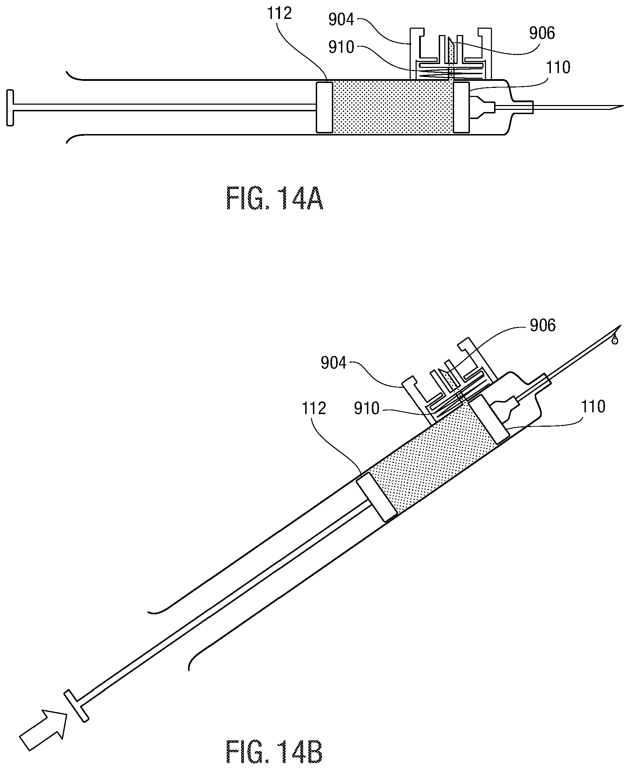

In operation, as shown in FIGS. 13A-13B and FIGS. 14A-14B, the auto-stop syringe is coupled to the support platform and the drive assembly of the motorized injection system. Next, the vial 902 is snapped into the receptacle 904 of the rapid fill port 900, which forces the sliding fill puncture element casing away from the side port of the fill puncture element. The fill puncture element of the rapid fill port then penetrates through the stopper of the vial to fluidly connect the internal volume of the vial with the syringe barrel through the side port of the fill puncture element. The fill needle of the rapid fill port then penetrates through the stopper of the vial to fluidly connect the internal volume of the vial 902 with the syringe barrel through the side port of the fill needle. This allows the therapeutic agent to flow from the vial 902 into the syringe barrel as the pushing plunger is withdrawn by the drive assembly. In some embodiments, a safety cap is provided on the puncture element of the injection system to fluidically seal the puncture element so that when the pushing sealing element is withdrawn, bubbles are not drawn into the syringe barrel as well.

Once the auto-stop syringe is loaded with a desired amount of the therapeutic agent, the vial can be removed from the receptacle of the rapid fill port, which allows the sliding fill needle casing to come up to seal the side port of the fill needle, which also seals the syringe barrel. The safety cap can be removed to allow fluid flow through the injection needle. The drive assembly can be activated to advance the pushing plunger until fluid appears at the tip of the injection needle indicating that the injection needle has been cleared of air. Then the auto-stop syringe is ready for use. This rapid fill port design can enable filling the auto-stop syringe with a therapeutic agent in a physician's office while maintaining sterility outside of a sterile facility.

In some embodiments, the auto-stop syringe of the present disclosure can be backfilled with the therapeutic agent. This can take place during the initial manufacturing of the syringe or at a physician's office immediately prior to use.

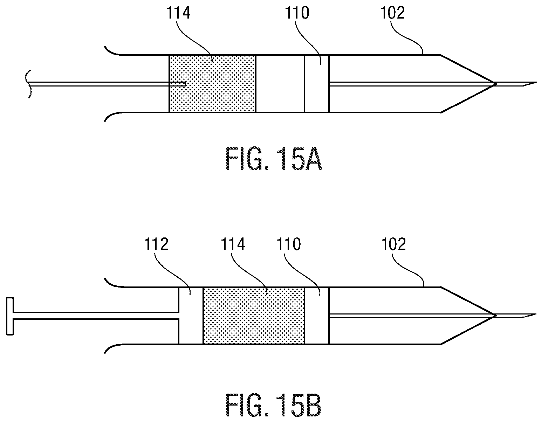

In some embodiments, as shown in FIGS. 15A-15B, the pushing plunger 112 can be removed, so the therapeutic agent 114 can be added to the syringe barrel 102 through the back of the syringe barrel. The pushing plunger can then be inserted and pushed toward the needle plunger 110 to remove any air in the injection needle.

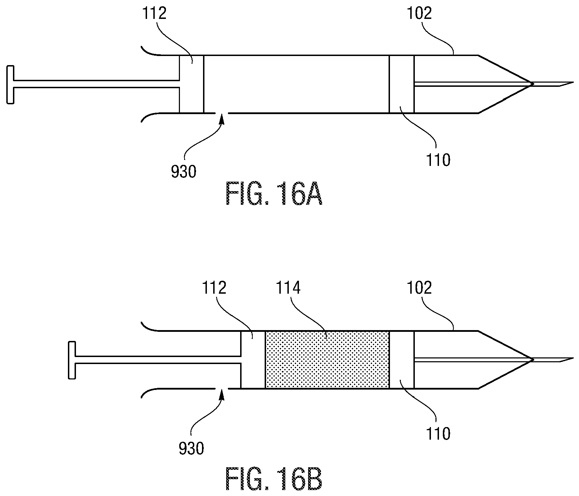

In some embodiments, as shown in FIGS. 16A-16B, a fill port 930 may be provided in the proximal region of the syringe barrel 102 distal of the pushing plunger 112. The therapeutic agent 114 can be added to the auto-stop syringe through this fill port 930 and then the pushing plunger 112 can be pushed past the fill port 930, so that the pushing plunger 112 seals the therapeutic fluid off from the fill port. In particular, the therapeutic agent can be added to the auto-stop syringe through the fill port using another sterile syringe/needle, while keeping needle side down (needle tip is blocked). In some embodiments, the total volume of the therapeutic agent can be about 80% of the volume between the plungers. Then, the pushing plunger can advance toward the needle plunger to remove air through the fill port. After the pushing plunger passes past the fill port, which blocks the fill port, the syringe can be flipped to bring the needle side up. Next, the pushing plunger is advanced further in the distal direction to release the remaining air out of the syringe barrel and the injection needle.

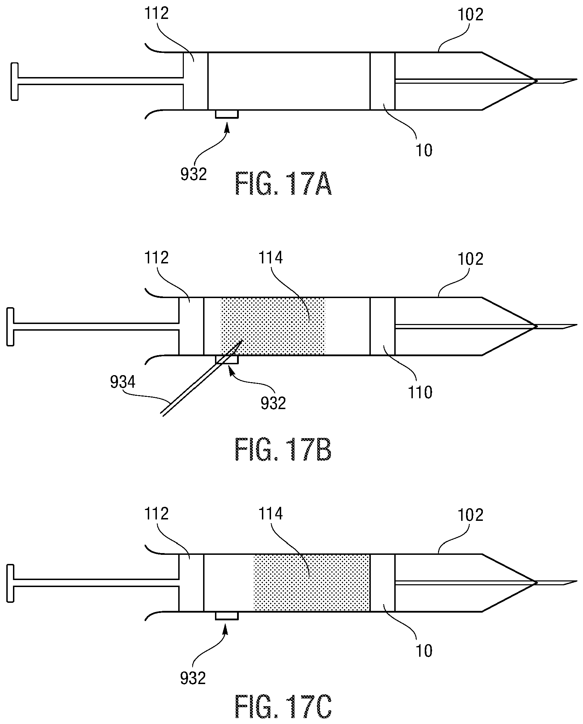

In some embodiments, as shown in FIGS. 17A-17C, the fill port 930 may be sealed using a self-sealing seal or polymer 932 (e.g. silicone rubber or polytetrafluoroethylene). In this way, the fill port can be filled with a separate, larger bore loading needle 934 of a standard syringe, while the syringe barrel of the auto-stop syringe can remain sealed throughout the process. When the loading needle is removed from the fill port, the fill port self-seals sufficiently to not leak under pressure applied by the pushing plunger during use.

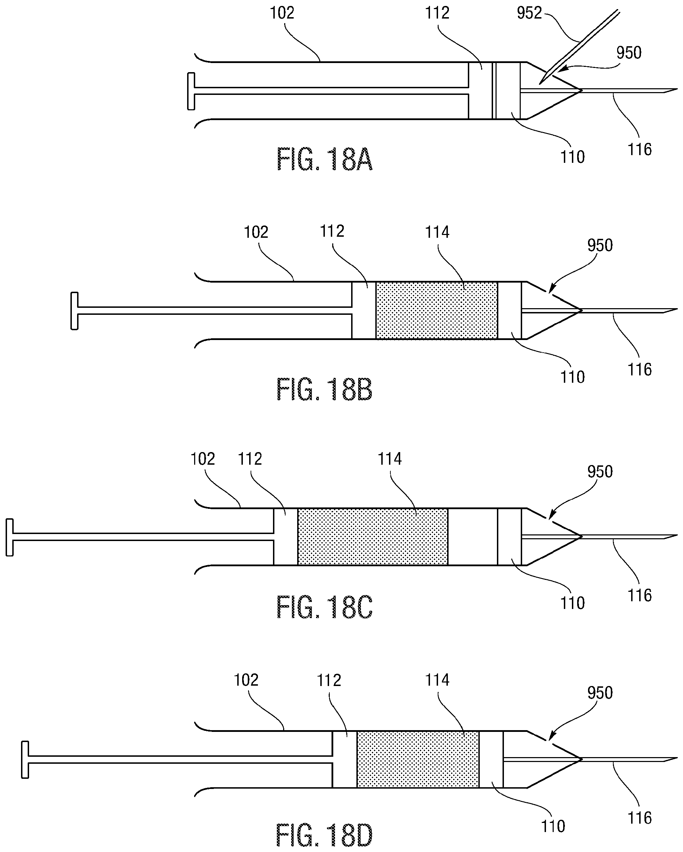

In some embodiments, as shown in FIGS. 18A-18D, a fill port 950 may be provided in the distal portion of the syringe barrel 102 in front of the needle plunger 110. This enables the user to access the needle plunger with a pushing tool 952 (e.g. a long, thin, rigid object that fits in the hole and is long enough to reach the outside). In this way, the injection needle can be extended outwards so that it can be pushed through an elastomeric vial stopper, then the therapeutic agent can be drawn into the syringe by withdrawing the pushing plunger. The pushing plunger can then be withdrawn further in the proximal direction, so that the needle plunger can be pushed back to its pre-insertion position within the syringe barrel.

### Uses of the Injection System

In some embodiments, the injection system of the present disclosure is used to deliver a viral gene delivery vector or vectors, including, but not limited to adeno-associated virus (AAV), a variant or serotype thereof, including but not limited to AAV serotypes 1-11, particularly AAV1, AAV2, AAV3, AAV4, AAV5, AAV6, AAV7, AAV8, AAV9, AAV10 and AAV11, and recombinant serotypes such as Rec2 and Rec3 to treat a genetic disorder of disease of the retina or choroid. AAV1, AAV2, AAV4, AAV5, AAV6, AAV7, AAV8, and AAV9 can all display tropism for retinal tissue, including retinal pigment epithelium and photoreceptors, as described in https://www.retinalphysician.com/issues/2020/special-edition-2020/vector-considerations-for-ocular-gene-therapy. Exemplary diseases can include, but not limited to wet age-related macular degeneration, dry age-related macular degeneration (AMD), glaucoma, choroideremia, and other heritable vision diseases and disorders. In some embodiments, the injection system is used to deliver a viral delivery vector or vectors, including, but not limited to AAV, or a variant thereof, to transfect retinal and/or choroidal cells, such as including, but not limited to, photoreceptors, pigmented cells, bipolar cells, ganglion cells, horizontal cells, and amacrine cells, vascular endothelial cells, vascular smooth muscle cells, nonvascular smooth muscle cells, melanocytes, fibroblasts, resident immunocompetent cells, with anti-vascular endothelial growth factor (anti-VEGF), and anti-vascular endothelial growth factor receptor (anti-VEGFR) gene that when transcribed produces an anti-VEGF protein or proteins for treating wet AMD. In some embodiments, the gene therapy compositions may also include a promoter for the gene of interest.

In some embodiments, the injection system is used to deliver gene therapies including, but not limited to small interfering ribonucleic acids (siRNAs), short hairpin ribonucleic acids (shRNAs), micro-ribonucleic acids (microRNAs), closed end-deoxyribonucleic acids (ceDNAs), polymer-DNA conjugates, or clustered regularly interspaced short palindromic repeats (CRISPR) and CRISPR-associated protein 9 (Cas9) systems, and variants thereof, and transcription activator-like effector nucleases (TALENs) and variants thereof, and zinc finger nucleases (ZFNs) and variants thereof and transposon-based gene delivery such as the Sleeping Beauty (SB), piggyBac (PB), Tol2 or variants thereof. These gene therapies can be packaged in viral vectors, non-viral vectors or nanoparticles.

In some embodiments, the injection system is used to deliver a viral gene delivery vector or vectors, non-viral gene delivery systems or other gene therapies achieves a transfection efficiency of the retinal and/or choroidal cells of less than 0.001%, 0.01%, 0.1%, 1%, 3%, 5%, 10%, 25%, 50%, 75% or 90%.

In some embodiments, the injection system is used to deliver a small or large molecule therapy targeted against VEGF or VEGFR, such as including, but not limited to, zivaflibercept, pazopanib, bevacizumab, cabozantinib, sunitinib, sorafenib, axitinib, regorafenib, ponatinib, cabozantinib, vandetanib, ramucirumab, lenvatinib, and bevacizumab.

In some embodiments, the injection system is used to deliver a gene therapy that targets, replaces, inhibits, or promotes one or more of the following genes to impart a therapeutic effect for a hereditary ocular disease or disorder including, but not limited to, MTP, HGD, SLC16A2, POLG, ALMS1, FGFR2, PRPS1, APTX, ATM, DNMT1, TGFBI, ACTB, FGFR2, BEST1, CYP4V2, NOD2, FOXL2, ABCC9, ERCC6, CYP27A1, CHS1, SH3BP2, HDAC6, CHM, SLC9A6, NSDHL, OPN1MW, OPN1LW, OPN1SW, KERA, IGBP1, OPA3, UGT1A1, FGFR2, FGFR3, ATP6V0A2, CTNS, EFEMP1, SALL4, ADAMTSL4, FBN1, ADAMTSL4, NR2E3, TGFBI, GLA, IKBKAP, LCAT, GALK1, GALT, GBA, GLB1, PORCN, TGFBI, OAT, ENG, CBS, MBTPS2, IKBKG, CNNM4, ATRX, GALC, TGFBI, HADHA, OCRL1, PLP1, B3GALTL, PAH, ARX, LOXL1, TGFBI, PQBP1, RB1, IDUA, IDS, SGSH, NAGLU, HGSNAT, GNS, GALNS, GLB1, ARSB, GUSB, FGFR3, LMX1B, NHS, STAC3, NF1, NF2, NF1, MT-ATP6, NDP, RP1L1, GPR143, PABN1, HEXB, UBIAD1, AGK, RAIL HBB, TIMP3, ATP2B3, ABCA4, ELOVL4, PROM1, GNAQ, SUOX, NAA10, BCOR, SOX2, OTX2, BMP4, HCCS, STRA6, VAX1, RARB, HMGB3, MAB21L2, RBM10, HEXA, TGFBI, SHOX, TAT, PTEN, VHL, VCAN, NF1, ZC4H2, ATP7B, CNGA3, CNGB3, JAG1, NOTCH2, PAX6, ELP4, FOXE3, PITX3, PITX2, FOXC1, CHD7, SEMA3E, ERCC6, ERCC8, CYP1B1, MYOC, MYOC, CYP1B1, FGFR1, FGFR2, FGFR1, FGFR2, NDN, SNRPN, PHYH, PEX7, CREBBP, EP300, OPA1, OPTN, SAG, GRK1, TWIST1, FGFR2, GPC3, OFD1, TSC1, TSC2, PRPH2, BEST1, WFS1, CISD2, COL4A5, COL4A4, COL4A3, UBE3A, CDKLS, MECP2, PTCH1, PTCH2, SUFU, NSD1, H19, KCNQ1OT1, CDKN1C, OPN1LW, OPN1MW, EYA1, SIX1, SIX5, KIF21A, PHOX2A, ARIX, TUBB3, SMC1A, HDAC8, COL5A1, COL5A2, COL3A1, TNXB, OPTN, ASB10, WDR36, MTND1, MTND4, MTNDS, MTND6, PAX6, PITX2, CYP1B1, FOXCl, DMPK, ZNF9, CNBP, NPC1, NPC2, SMPD1, TYR, OCA2, TYRP1, or SLC45A2, MC1R, COL1A1, COL1A2, CRTAP, LEPRE1, NPHP1, NPHP4 SDCCAG8, WDR19, CEP290, IQCB1, HESX1, OTX2, SOX2, COL2A1, COL11A1, COL11A2, COL9A1, COL9A2, MYO7A, USH2A, EDN3, EDNRB, MITF, PAX3, SNA12, SOX10, ADAMTS10, FBN1, LTBP2, XPA, XPC, ERCC2, ERCC3, and POLH.

In some embodiments, the delivery system of the present disclosure may be used to deliver gene therapy to treat age-related macular degeneration (AMD) or diabetic macular edema (DME). In some embodiments, the delivery system of the present disclosure is used for suprachoroidal (SCS) delivery of a composition comprising a AAV vector including one or more genes to block VEGFR-2, optionally with a CAG promoter. In some embodiments, other suitable promoters include, but are not limited to, human bestrophin (hVMD2), cytomegalovirus (CMV), SV40, mGluR6, CB7, UbiC, RZ, RedO, Rho and Best1. In some embodiments, such system may include a 25-34 gauge puncture element with a polypropylene or glass syringe and fluoropolymer, silicone or rubber for the pushing sealing element stopper and floating sealing element stopper. In some embodiments, about 80-120 (for example, 100) microliters of such gene therapy composition can be delivered over 5-60 seconds. In some embodiments, the puncture element may have a bevel length less than 2mm, less than 1mm or less than 0.5mm. The bevel angle can be greater than 15 degrees, greater than 30 degrees, or even greater than 45 degrees. In some embodiments, the puncture element can be 25 gauge and higher, 27 gauge and higher, or 30 gauge or higher. In some embodiments the needle has a secondary bevel to lower cutting forces.

In some embodiments, the delivery system is utilized to deliver small or large molecule injection agents such as, anti-VEGF drugs including, but not limited to, bevacizumab, ranibizumab, aflibercept, Ramucirumab, disintegrins, anti-prostaglandins, tryptophanyltRNAsynthetase-derived polypeptides, Inosine monophosphate dehydrogenase (IMPDH) inhibitors and anti-PDGF to treat AMD; and corticosteroids to treat uveitis, chorioretinitis, or other inflammatory eye diseases; botulinum toxin for various ocular applications; tyrosine kinase inhibitors (such as Vandetanib, Axitinib, Pazopanib, Sunitinib, Sorafenib) to treat pterygium, dry eye or AMD; levo-betaxolol, or other betaadrenoceptor antagonists and 5-HT1A agonists to treat retinal pathologies.

In some embodiments, the injection system is used to deliver small molecule Wnt inhibitors to decrease angiogenesis. These small molecular Wnt inhibitors can include indazole-3-carboxamide compound or analogs thereof (WO2013040215A1), y-diketones or salts or analogs thereof (WO2014130869A1), azaindazole compound or analogs (e.g. 3-(1h-benzo[d]imidazol-2-yl)-1h-pyrazolo[3,4-c]pyridine) thereof (WO2016040180A1), N-(5-(3-(7-(3-fluorophenyl)-3H-imidazo[4,5-c]pyridin-2-yl)-1H-indazol-5-yl)pyridin-3-yl)-3-methylbutanamide, including amorphous and polymorph forms thereof (WO2017210407A1), Isoquinolin-3-yl carboxamides or salt or analogs and including amorphous and polymorph forms thereof (WO2017189823A2), Diazanaphthalen-3-yl carboxamides or salt or analogs and including amorphous and polymorph forms (US20190127370A1), 6-(5-membered heteroaryl)isoquinolin-3-yl-(5-membered heteroaryl) carboxamides or salt or analogs and including amorphous and polymorph forms (WO2019084496A1), 6-(6-membered heteroaryl & aryl)isoquinolin-3-yl carboxamides or salt or analogs and including amorphous and polymorph forms (US20190125740A1), 3-(3h-imidazo[4,5-b]pyridin-2-yl)-1h-pyrazolo[3,4-b]pyridine (US20190119303A1), Wnt inhibitors containing an indazole core or salt or analogs and including amorphous and polymorph forms (WO2013151708A1), lh-pyrazolo[3,4-b]pyridines or salt or analogs and including amorphous and polymorph forms (WO2013166396A2), 2-(1h-indazol-3-yl)-3h-imidazo[4,5-b]pyridine or salt or analogs and including amorphous and polymorph forms (US20190055238A1), f3-diketone, y-diketone or y- hydroxyketone or salts or analogs thereof (WO2012024404A1), 3-(benzoimidazol-2-yl)-indazole inhibitors or salt or analogs and including amorphous and polymorph forms (US10183929B2), 3-(1h-imidazo[4,5-c]pyridin-2-yl)-1h-pyrazolo[3,4-b]pyridine or salt or analogs and including amorphous and polymorph forms (US20180325910A1), 1H-pyrazolo [3,4-b] pyridines or salt or analogs and including amorphous and polymorph forms (CY-1119844-T1), 3-(1h-imidazo[4,5-c]pyridin-2-yl)-1h-pyrazolo[3,4-c]pyridine or salt or analogs and including amorphous and polymorph forms (US-2018250269-A1), N-(5-(3-(7-(3-fluorophenyl)-3H-imidazo[4,5-c]pyridin-2-yl)-1H-indazol-5-yl)pyridin-3-yl)-3-methylbutanamide or salt or analogs and including amorphous and polymorph forms, (US20180133199A1), indazole-3-carboxamides or salt or analogs and including amorphous and polymorph forms (US-2018185343-A1), 3-(3h-imidazo[4,5-b]pyridin-2-yl)-1h-pyrazolo[3,4-c]pyridine or salt or analogs and including amorphous and polymorph forms (US-2018201624-Al), 2-(1h-indazol-3-yl)-1h-imidazo[4,5-c]pyridine or salt or analogs and including amorphous and polymorph forms (US-2018215753-A1), 3-(3H-imidazo[4,5-C]pyridin-2-yl)-1H-pyrazolo[3,4-C]pyridine or salt or analogs and including amorphous and polymorph forms (US-10052331-B2), 5-substituted indazole-3-carboxamides or salt or analogs and including amorphous and polymorph forms (US-2018127377-A1), 3-(3H-imidazo[4,5-C]pyridin-2-yl)-1 H-pyrazolo[4,3-B]pyridines or salt or analogs and including amorphous and polymorph forms (US-10188634-B2), 3-(1H-imidazo[4,5-C]pyridin-2-yl)-1H-pyrazolo[4,3-B]pyridines or salt or analogs and including amorphous and polymorph forms (US-10195185-B2), 3-(1h-pyrrolo[2,3-b]pyridin-2-yl)-1h-indazoles or salt or analogs and including amorphous and polymorph forms (WO-2017024021-A1), 3-(1h-pyrrolo[2,3-c]pyridin-2-yl)-1h-pyrazolo[3,4-c]pyridines or salt or analogs and including amorphous and polymorph forms (WO-2017023975-A1), 3-(1h-indol-2-yl)-1h-pyrazolo[3,4-b]pyridines or salt or analogs and including amorphous and polymorph forms (US-2018214428-A1), 3-(1h-pyrrolo[3,2-c]pyridin-2-yl)-1h-indazoles or salt or analogs and including amorphous and polymorph forms (US-2018221350-A1), 3-(1h-indol-2-yl)-1h-indazoles or salt or analogs and including amorphous and polymorph forms (WO-2017023986-A1), 3-(1H-pyrrolo[2,3-B]pyridin-2-yl)-1H-pyrazolo[4,3-B]pyridines or salt or analogs and including amorphous and polymorph forms (US-10206909-B2), 3-(1h-pyrrolo[3,2-c]pyridin-2-yl)-1h-pyrazolo[4,3-b]pyridines or salt or analogs and including amorphous and polymorph forms (WO-2017024003-Al), 3-(1h-pyrrolo[3,2-c]pyridin-2-yl)-1h-pyrazolo[3,4-b]pyridines or salt or analogs and including amorphous and polymorph forms (US-2018221341-A1), 3-(3h-imidazo[4,5-b]pyridin-2-yl)-1h-pyrazolo[4,3-b]pyridines or salt or analogs and including amorphous and polymorph forms (WO-2017024015-A1), 3-(1h-pyrrolo[2,3-c]pyridin-2-yl)-1h-pyrazolo[3,4-b]pyridines or salt or analogs and including amorphous and polymorph forms (US-2018221352-Al), 3-(1H-pyrrolo[3,2-C]pyridin-2-YL)-1H-pyrazolo[3,4-C]pyridines or salt or analogs and including amorphous and polymorph forms (US-10206908-B2).

In some embodiments, the injection system is utilized to deliver suspensions of injection agents including microencapsulated agents, nanoencapsulated agents, pure protein nanoparticles and poorly water-soluble or water-insoluble agents.

In some embodiments, the injection agent or encapsulated injection agent is delivered with a residence time extending matrix. The matrix can consist of reverse thermally responsive hydrogels, self-assembling hydrogels, bioadhesive polymer networks, hydrogels, fibronectin-containing hydrogels, enzyme-responsive hydrogels, ultrasound sensitive hydrogels, pH-sensitive hydrogels, carbohydrates, two or more component hydrogels, and multi-component double network hydrogels.

In some embodiments, the injection agent is delivered via the injection system with following a permeation enhancer such as including, but not limited to, dimethylsulfoxide (DMSO), collagenases, elastases, proteases, papain, bromelain, peptidases, lipases, alcohols, polyols, short chain glycerides, amines, amides, cyclodextrins, fatty acids, pyrrolidones, Cyclopentadecalactone, Sodium N-[8-(2-hydroxylbenzoyl)amino] caprylate (SNAC), 8-(N-2-hydroxy-5-chloro-benzoyl)-amino-caprylic acid (5-CNAC), Sodium caprate, Sodium caprylate, omega 3 fatty acid, protease inhibitors, alkylglycosides, chitosan, Dodecyl-2-N,N-dimethylamino propionate (DDAIP), N-methyl-2-pyrrolidone (NMP), azones, sulfoxides, surfactants, benzylalkonium choride, saponin, bile salts, bile acids, cell penetrating peptides, polyarginine, low molecular weight protamine, polyserine, capric acid, gelucires, semifluorinated alkanes, terpenes, phospholipids, chelators, Ethylenediamine Tetraacetic acid (EDTA), citrate, crown ethers and combinations thereof.

In some embodiments, the injection agent having one or more therapeutic formulations is delivered via the injection system with or following administration of one or more vasoconstrictive agents to reduce efflux of the injection agent via the choroidal blood vessels, including, but not limited to 25I-NBOMe, Amphetamines, AMT, Antihistamines, Caffeine, Cocaine, Dopamine, Dobutamine, DOM, LSA, LSD, Methylphenidate, Mephedrone, Norepinephrine, Oxymetazoline, Phenylephrine, Propylhexedrine, Pseudoephedrine, Stimulants, Serotonin 5-hydroxytryptamine agonists, triptans and Tetrahydrozoline hydrochloride. In some embodiments, these agents may be administered using the injection system of the present disclosure into the SCS or via an intravitreal injection using a standard syringe. The vasoconstrictive agents can be delivered before, simultaneously, or after the administration of the one or more therapeutic formulations.

In some embodiments, the injection agent delivered via the injection system achieves SCS coverage in excess of 20%, 40%, 60% or 80%.

In some embodiments, the injection agent delivered via the injection system with or without one or more vasoconstrictive agents to reduce efflux of the injection agent via the choroidal blood vessels achieves SCS coverage in less than 180, 120, 60, 30 or 15 minutes.

In some embodiments, the injection agent delivered via the injection system has a retention time within the SCS of less than 180, 120, 60, 30, 15, 10 or 5 minutes.

In some embodiments, the injection agent is delivered via the injection system in less than 500, 400, 300, 200 or 100 microliters.

In some embodiments, the injection agent is delivered via the injection system in concentrations less than 80%, 60%, 40% 20%, 10%, 5%, 2.5% or 1%.

In some embodiments, the percent dosage of the injection agent delivered via the injection system delivered to the subretinal space is less than 80%, 60%, 40% 20%, 10%, 5%, 2.5% or 1%.

In some embodiments, the injection agent delivered via the injection system is dosed at least once every 10 years, once every 5 years, once every 2 years, once every 1 year, once every 6 months, once every 3 months, once a month or once a week.

In some embodiments, the injection system is used to deliver one or multiple injection agents to treat one or more of the ocular causes or effects of the following diseases including, but not limited to, Abetalipoproteinemia (Bassen-Kornzweig Syndrome), Alkaptonuria, Allan-Herndon-Dudley Syndrome, Alpers Syndrome, Alstrom Syndrome, Apert Syndrome, Arts Syndrome (Mental Retardation, X-Linked, Syndromic 18), Ataxia-Oculomotor Apraxia Syndrome, Ataxia Telangiectasia (Louis-Bar Syndrome), Autosomal Dominant Cerebellar Ataxia Deafness and Narcolepsy (ADCADN), Avellino Corneal Dystrophy (Combined Granular-Lattice Corneal Dystrophy), Baraitser-Winter Syndrome 1, Beare-Stevenson Syndrome, Best Macular Dystrophy, Bietti Crystalline Corneoretinal Dystrophy, Blau Syndrome, Blepharophimosis, Ptosis, and Epicanthus Inversus (BPES), Cantu Syndrome, Cerebrooculofacioskeletal Syndrome, Cerebrotendinous Xanthomatosis, Chediak-Higashi Syndrome, Cherubism, Chondrodysplasia with Platyspondyly, Distinctive Brachydactyly, Hydrocephaly, and Microphthalmia, Choroideremia, Christianson Syndrome, CK Syndrome, Colorblindness, Deutan, Colorblindness, Protan, Colorblindness, Tritanopic, Cornea Plana, Corpus Callosum, Agenesis of, with Mental Retardation, Ocular Coloboma, and Micrognathia, Costeff Syndrome, Crigler-Najjar, Crouzon Syndrome, Crouzon Syndrome with Acanthosis Nigricans (Crouzonodermoskeletal Syndrome), Cutis Laxa, Debre Type, Cystinosis, Doyne Honeycomb Dystrophy (Malattia Leventinese), Duane-Radial Ray Syndrome, Ectopia Lentis et Pupillae, Ectopia Lentis, Familial, Ectopia Lentis, Isolated, Enhanced S-Cone Syndrome, Epithelia Basement Membrane Corneal, Dystrophy (Map-Dot-Fingerprint Corneal Dystrophy), Fabry Disease (Hereditary, Dystopic Lipidosis), Familial Dysautonomia, Fish-Eye Disease, Galactokinase Deficiency, Galactosemia, Gaucher's Disease, GM1-Gangliosidosis, Type I, GM1-Gangliosidosis, Type II, GM1-Gangliosidosis, Type III, Goltz Syndrome, Granular, Corneal Dystrophy (Groenouw Type I), Gyrate Atrophy, Hereditary Hemorrhagic Telangietasia (Osler-Rendu-Weber Disease), Homocystinuria, IFAP Syndrome with or without Bresheck Syndrome, Incontinentia Pigmenti (Bloch-Sulzberger Syndrome), Jalili Syndrome, Juberg-Marsidi Syndrome, Krabbe Disease, Lattice Corneal Dystrophy, LCHAD (Long-Chain 3-Hydroxyacyl-Coa Dehydrogenase) Deficiency, Lowe, Pelizaeus-Merzbacher, Peters-Plus Syndrome (Krause-Kivlin Syndrome), Phenylketonuria, Proud Syndrome, Pseudoexfoliation Syndrome, Reis-Bucklers Corneal Dystrophy, Renpenning Syndrome (Mental Retardation, X-Linked, Renpenning Type), Retinoblastoma, Retinoschisis, Juvenile X Linked, Russell-Silver Syndrome, Mucopolysaccharidosis Type IH (Hurler Syndrome), Mucopolysaccharidosis Type lIDS (Hurler-Scheie Syndrome), Mucopolysaccharidosis Type IS (Scheie Syndrome), Mucopolysaccharidosis Type II (Hunter Syndrome), Mucopolysaccharidosis Type IIIA (Sanfilippo Syndrome A), Mucopolysaccharidosis Type IIIB (Sanfilippo Syndrome B), Mucopolysaccharidosis Type IIIC (Sanfilippo Syndrome C), Mucopolysaccharidosis Type IIID (Sanfilippo Syndrome D), Mucopolysaccharidosis Type IVA (Morquio Syndrome A), Mucopolysaccharidosis Type IVB (Morquio Syndrome B), Mucopolysaccharidosis Type VI (Maroteaux-Lamy Syndrome), Mucopolysaccharidosis Type VII (Sly Syndrome), Muenke Syndrome, Nail-Patella Syndrome, Nance-Horan Syndrome Native American Myopathy, Neurofibromatosis Type I, Neurofibromatosis Type II, Neurofibromatosis-Noonan Syndrome, Neuropathy, Ataxia, and Retinitis, Pigmentosa (NARP), Norrie Disease, Occult Macular Dystophy, Ocular Albinism, Oculopharyngeal Muscular Dystrophy, Sandhoff Disease (GM2-Gangliosidosis, Type II), Schnyder Corneal Dysrophy, Sengers Syndrome, Smith-Magenis Syndrome, (Chromosome 17p11.2 Deletion Syndrome), Sickle Cell Anemia, Sorsby Fundus Dystrophy, Spinocerebellar Ataxia, X-Linked 1, Stargardt Disease/Fundus, Flavimaculatus, Sturge-Weber Syndrome, Sulfocysteinuria (Sulfite Oxidase Deficiency), Syndromic Microphthalmia 1 (Lenz Microphthalmia Syndrome), Syndromic Microphthalmia 2 (Oculofaciocardiodental Syndrome), Syndromic, Microphthalmia 3 (Microphthalmia and Esophageal Atresia Syndrome), Syndromic Microphthalmia 5, Syndromic Microphthalmia 6, Syndromic Microphthalmia 7, (Midas Syndrome), Syndromic Microphthalmia 9 (Matthew-Wood Syndrome), Syndromic Microphthalmia 11, Syndromic Microphthalmia 12, Syndromic Microphthalmia 13, Syndromic Microphthalmia 14, Tarp Syndrome, Tay-Sachs Disease (GM2-Gangliosidosis, Type I), Thiel-Behnke Corneal Dystrophy, Turner Syndrome, Tyrosinemia, Type II, Vacterl Association with Hydrocephalus, Von Hippel-Lindau Syndrome, Wagner Syndrome, Watson Syndrome, Wieacker-Wolff Syndrome, Wilson Disease, Achromatopsia, Alagille Syndrome, Aniridia, Anterior Segment Mesenchymal Dysgenesis, Axenfeld-Rieger Syndrome, Charge Syndrome, Cockayne Syndrome, Glaucoma, Congenital, Glaucoma, Open Angle Juvenile Onset, Jackson-Weiss Syndrome, Pfeiffer Syndrome, Prader-Willi Syndrome, Ref Sum Disease, Rubinstein-Taybi Syndrome, Normal-Tension Glaucoma, Oguchi Disease, Saethre-Chotzen Syndrome, Simpson-Golabi-Behmel Syndrome, Tuberous Sclerosis, Vitelliform Macular Dystrophy, Adult-Onset, Wolfram Syndrome, Alport Syndrome, Angelman Syndrome, Bardet Biedl Syndrome, Basal Cell Nevus Syndrome, Beckwith-Wiedemann Syndrome, Blue-Cone Monochromacy, Branchiootorenal Syndrome, Charcot-Marie-Tooth Disease, Cone-Rod Dystrophy, Congenital Disorder of Glycosylation, Congential Fibrosis of Extraocular Muscles, Congenital Nystagmus, Congenital Stationary Night Blindness, Cornelia de Lange Syndrome, Dyskeratosis Congenita, Ehlers-Danlos Syndrome, Fuch's Endothelial Corneal Dystrophy, Glaucoma, Open Angle Adult Onset, Hermansy-Pudlak Syndrome, Joubert Syndrome, Kearns-Sayre Syndrome, Leber Congenital Amaurosis, Leber Hereditary Optic Neuropathy, Leigh Syndrome, Peters' Anomaly Retinitis Pigmentosa, Muscular Dystrophy-Dystroglycanopathy, Myotonic Dystrophy, Niemann-Pick Disease, Noonan Syndrome, Neuronal Ceroid Lipofuscinosis, Oculocutaneous Albinism, Optic Atrophy, Oral-Facial-Digital Syndrome, Osteogenesis Imperfecta, Senior-Loken Syndrome, Septic-Optic Dysplasia (de Morsier Syndrome), Spastic Paraplegia, Stickler Syndrome, Treacher Collins Syndrome, Usher Syndrome, Waardenburg Syndrome, Weill-Marchesani Syndrome, and Xeroderma Pigmentosum.

In some embodiments, multiple injections may be performed over time to allow continuation of therapy. The injection of therapeutic may be accompanied by another agent that enables multiple deliveries. For e.g. AAV delivery is limited by immune response to AAV which usually limits the AAV usage to a single time treatment, a limitation commonly associated with intravitreal injection, and while sub-retinal injection is immune privileged, the damaged and diseased retina does not tolerate multiple injections without trauma. Another agent (such as ImmTOR ) that suppresses this response can be injected prior, in combination, or after the AAV injection to mitigate the immune response and enable AAV therapy at multiple time points. This allows one to titrate the dose to patient response as necessary.

In some embodiments, the route of administration is by injection into the SCS. In some embodiments, the genetic disease or disorder is diagnosed by gene sequencing such as including, but not limited to, Sanger sequencing, next generation sequencing, high-throughput screening, exome sequencing, Maxam-Gilbert sequencing, chain-termination methods, shotgun sequencing, Bridge polymerase chain reaction, single molecule real-time sequencing, ion torrent sequencing, pyrosequencing, sequencing by synthesis, combinatorial probe anchor synthesis, sequencing by ligation and nanopore sequencing. In some embodiments, the ocular disease or disorder is diagnosed by an eye exam, an ophthalmoscope, ocular coherence tomography, retinal scanning, fluorescein staining, conjunctival staining, color vision testing, optic disc imaging, nerve fiber layer analysis, corneal topography, electro-diagnostic testing, fluorescein angiography, photography of the eye, specular microscopy, visual field testing, ultrasound of the eye and combinations thereof.

In some embodiments, a patient presents with elevated intraocular pressure and is diagnosed with early stage juvenile primary open angle glaucoma before significant optic nerve damage has occurred after being examined with an ophthalmoscope. A blood sample is drawn and sent for genetic testing, which determines that the patient has a mutation in the olfactomedin domain of his myocilin (MYOC) gene, mutation Y437H, that is likely implicated in causing the disease, leading to a diagnosis of myocilin-associated primary open angle glaucoma.

The patient is then treated by dosing with the injection system, administering microRNA complementary to the first 22 bases of mRNA for the MYOC gene formulated in aqueous solution of a self-assembling hydrogel with betacyclodextrin and EDTA as permeation enhancers. Prior to use, the injection is stored as a lyophilized powder in separate vials from the diluent. Following injection, the hydrogel self-assembles in the SCS after delivery providing sustained delivery of the microRNA that suppresses myocilin expression, leading to a reduced accumulation of myocilin in the trabecular meshwork, resulting in reduced intraocular pressure, thereby reducing the probability of sustaining optic nerve damage for the patient.

In another specific embodiment, a male child presents with night blindness and on exam is found to have reduced visual field and some retinal degeneration. A blood sample is drawn and sent for genetic testing, which determines that the patient has a mutation in his CHM gene, containing part or the entirety of the CHM gene sequence as described, for example, in https://www.uniprot.org/uniprot/P24386, which encodes RAB escort protein 1 (REP1), which supports a diagnosis of early stage choroideremia.

The patient is then treated by dosing with the injection system, in which lyophilized AAV2 vector containing a retinal specific promoter, derived from the rhodopsin kinase (RK) promoter gene expressed in rods and cones, connected to the human CHM gene, has been reconstituted with its aqueous diluent prior to injection. On reconstitution, the injection agent solution contains approximately 1013 AAV vectors per milliliter. Once injected, the RK promoter and human CHM gene will be stably transfected into photoreceptor cells, where the corrected form of REP 1 will be expressed, treating the patient's choroideremia.

In another specific embodiment, an elderly patient presents with central vision defects. On routine retinal examination, drusen are detected. Fluorescein angiography demonstrates leaky choroidal vasculature, confirmed by the presence of sub-retinal fluid accumulation observed on optical coherence tomography (OCT). The patient is diagnosed with early stage neovascular age-related macular degeneration (AMD).

The patient is then treated by dosing with the injection system, in which 21-24 nucleotide short interfering RNA (siRNA) sequences complementary to portions the mRNA of one or more of the following alone or in combination of, vascular endothelial growth factor (VEGF), any of its sub-types including, but not limited to VEGF-A, VEGF-A121, VEGF-A165, VEGF-A189, VEGF-A206 VEGF-B, VEGF-C, VEGF-D, VEGF receptors (VEGFRs), VEGFR-1, VEGFR-2, VEGFR-3, NOTCH regulated ankyrin repeat protein (NRARP), and other angiogenesis promoting proteins encoding genes. The siRNA is delivered in a suspension of liposomal carriers. Following delivery, the siRNA knocks down expression of the angiogenesis promoting protein or proteins thereby preventing additional choroidal capillary growth and causing capillary regression yielding reduced choroidal capillary retinal and macular invasion and improved central vision. In a specific embodiment, the siRNA is targeted to knock down VEGFR-2, which has a gene sequence or isoforms thereof as described in https ://www.uniprot.org/uniport/P35968,

In another specific embodiment, a patient diagnosed with neovascular AMD or diabetic retinopathy is treated by dosing with the injection system, in which an AAV vector, or other transfection vector, contains a gene that when transcribed produces an RNA sequence that is complementary to at least a portion of the mRNA that is translated into VEGFR-2. In delivering this gene therapy to the SCS, the choroidal capillaries, also referred to as choriocapillaris, contact the delivered therapeutic targeted at transfecting those cells expressing VEGFR-2. On transfection, the siRNA or shRNA vectors that are transcribed knock down or knock out VEGFR-2 production thereby reducing neovascularization to treat AMD or diabetic retinopathy.

In some embodiments, the physician may be presented with a suprachoroidal injection assembly or kit, which includes (1) a volume of the injection agent comprising one or more therapeutic agent formulations, i.e. active agent formulations, for example, containing an effective amount of an agent useful for treating a condition of an eye of a patient; (2) an injection system as described above and (3) optionally, an injector to facilitate ejection of the injection agent into and through the injection system membrane.

As described earlier, the agent formulation can comprise of various forms, such as solutions and suspensions of various viscosity. The entire kit is sterile including the formulation, injection system, and facilitating injector.

In some embodiments, the total volume of the active agent formulation to be injected in the suprachoroidal space is preferably in the range of approximately 0.01-0.5 mL. In some embodiments, the active agent may be provided in a lyophilized form and an accompanying diluent to create the suspension at the time of injection. In some embodiments, the active agent may be premixed. In some embodiments, the injection system may be prefilled with premixed formulation. In some embodiments, the user may fill the injection system immediately prior to administering the therapeutic formulation to the patient. In some embodiments, the injection system may contain multiple chambers with frangible separation. In some embodiments, the puncture element has initial penetrating length of 0.01 to 3 mm and the puncture element extends further while performing injection. In some embodiments, the injection system and injection facilitator can be preassembled with prefilled formulation and ready for use without any further assembly. In some embodiments, entire kit is packaged in a single pouch/tray to maintain sterility. In some embodiments, where components are packaged separately or in a combination. In some embodiments, the kit is sterilized together or separately by one of the sterilization methods including but not limited to autoclave, ethylene oxide, gamma radiation etc.

In some embodiments, where the components are present in a secondary package. In some embodiments, the kit is stored as a set at low enough temperature to extend the life of the active pharmaceutical agent. In some embodiments, the formulation is stored at low temperature separately while the rest of kit is stored at room temperature.

### Computer Systems for Use with the Injection Systems

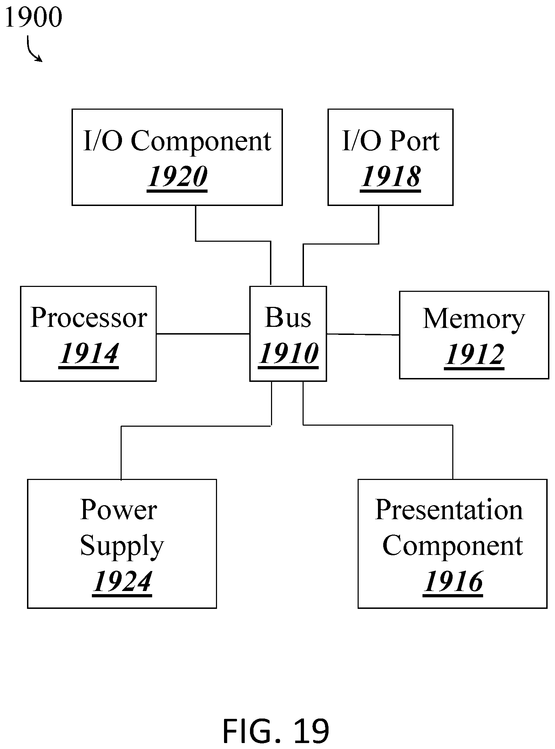

The systems of the present disclosure can include a controller for controlling the operation of the injection systems of the present disclosure. In some embodiments, such controller may be a computer system to collect and analyze the sensor information used by the system to control the injection assembly. Any suitable computing systems can be used to implement the computing devices and methods/functionality described herein and be converted to a specific system for performing the operations and features described herein through modification of hardware, software, and firmware, in a manner significantly more than mere execution of software on a generic computing device, as would be appreciated by those of skill in the art. One illustrative example of such a computing device 1900 is depicted in FIG. 19. The computing device 1900 is merely an illustrative example of a suitable computing environment and in no way limits the scope of the present invention. A "computing device," as represented by FIG. 19, can include a "workstation," a "server," a "laptop," a "desktop," a "hand-held device," a "mobile device," a "tablet computer," or other computing devices, as would be understood by those of skill in the art. Given that the computing device 1900 is depicted for illustrative purposes, embodiments of the present invention may utilize any number of computing devices 1900 in any number of different ways to implement a single embodiment of the present invention. Accordingly, embodiments of the present invention are not limited to a single computing device 1900, as would be appreciated by one with skill in the art, nor are they limited to a single type of implementation or configuration of the example computing device 1900.

The computing device 1900 can include a bus 1910 that can be coupled to one or more of the following illustrative components, directly or indirectly: a memory 1912, one or more processors 1914, one or more presentation components 1916, input/output ports 1918, input/output components 1920, and a power supply 1924. One of skill in the art will appreciate that the bus 1910 can include one or more busses, such as an address bus, a data bus, or any combination thereof. One of skill in the art additionally will appreciate that, depending on the intended applications and uses of a particular embodiment, multiple of these components can be implemented by a single device. Similarly, in some instances, a single component can be implemented by multiple devices. As such, FIG. 19 is merely illustrative of an exemplary computing device that can be used to implement one or more embodiments of the present invention, and in no way limits the invention.

The computing device 1900 can include or interact with a variety of computer-readable media. For example, computer-readable media can include Random Access Memory (RAM); Read Only Memory (ROM); Electronically Erasable Programmable Read Only Memory (EEPROM); flash memory or other memory technologies; CDROM, digital versatile disks (DVD) or other optical or holographic media; magnetic cassettes, magnetic tape, magnetic disk storage or other magnetic storage devices that can be used to encode information and can be accessed by the computing device 1900.

The memory 1912 can include computer-storage media in the form of volatile and/or nonvolatile memory. The memory 1912 may be removable, non-removable, or any combination thereof. Exemplary hardware devices are devices such as hard drives, solid-state memory, optical-disc drives, and the like. The computing device 1900 can include one or more processors that read data from components such as the memory 1912, the various I/O components 1920, etc. Presentation component(s) 1916 present data indications to a user or other device. Exemplary presentation components include a display device, speaker, printing component, vibrating component, etc.

The I/O ports 1918 can enable the computing device 1900 to be logically coupled to other devices, such as I/O components 1920. Some of the I/O components 1920 can be built into the computing device 1900. Examples of such I/O components 1920 include a microphone, joystick, recording device, game pad, satellite dish, scanner, printer, wireless device, networking device, and the like.

## Claims

1. An injection system (10) comprising:
an injection assembly (14) comprising a syringe barrel (302) defining a lumen between a proximal end (302p) and a distal end (302d) and a second sealing element (304) moveably disposed within the lumen to dispense an injection agent from an injection chamber defined in the syringe barrel (302), and a puncture element (15) configured to deliver the injection agent into a space in a tissue of a patient, the tissue being less permeable to the injection agent than the space;
a support platform (12) configured to support the injection assembly (14) and anchor the injection assembly (14) relative to a site of injection;
a drive assembly (16) configured to operate the injection assembly (14);
one or more sensors configured to monitor one or more forces on the injection assembly (14); and
a controller in communication with the one or more sensors to receive information about the one or more forces on the injection system (10), and being configured to, based on the information, to operate the drive assembly (16) to advance the puncture element (15) through the tissue toward the space such that the injection agent remains in the injection chamber until the puncture element (15) fluidly connects the injection chamber with the space.

2. The injection system of claim 1, wherein the injection assembly (14) further comprises:
a first sealing element (306) moveably disposed within the lumen distal to the second sealing element (304), wherein the first sealing element (306) and the second sealing element (304) form a seal with the lumen and define the injection chamber between them, the puncture element (15) being in fluid communication with the injection chamber to deliver the injection agent from the injection chamber into the space in the tissue of the patient,
wherein the first sealing element (306) is a needle plunger and when a force is applied on the second sealing element (304) in a distal direction,
in response to a first opposing force as the puncture element (15) advances through the tissue, the first sealing element (306) moves in the distal direction to advance the puncture element (15) in the distal direction, without conveying the injection agent through the puncture element (15), and
in response to a second opposing force when the injection chamber is fluidly connected to the space, the first sealing element (306) remains stationary and the injection agent is conveyed from the injection chamber through the puncture element (15).

3. The injection system of claim 1wherein:
the injection assembly (14) comprises a first sealing element (306) moveably disposed within the lumen, the second sealing element (304) being distal to the first sealing element (306) to define an injection chamber, and the puncture element (15) is fluidly connected to the injection chamber;
the first sealing element (306) is a needle plunger;
the drive assembly (16) is configured to translate one or both of the syringe barrel (302) or the second sealing element (304) relative to the support platform (12);
one or more sensors configured to monitor one or more forces on the injection assembly (14); and
the controller is configured such that when the drive assembly (16) translates the second sealing element (304) in a distal direction,
in response to a first opposing force as the puncture element (15) advances through the tissue, the first sealing element (306) moves in the distal direction to advance the puncture element (15) in the distal direction, without conveying the injection agent through the puncture element (15), and
in response to a second opposing force when the injection chamber is fluidly connected to the space, the first sealing element (306) remains stationary and the injection agent is conveyed from the injection chamber through the puncture element (15).

4. The injection system of any one of claims 1 to 3, wherein the drive assembly (16) is configured to translate independently of one another the syringe barrel (302) and the second sealing element (304) relative to the support platform (12).

5. The injection system of any one of claims 1 to 3, wherein the drive assembly (16) is linked to the second sealing element (304) to apply the force on the second sealing element (304) to translate the second element in the distal direction.

6. The injection system of any one of claims 1 to 3, wherein the drive assembly (16) comprises a linear actuator linked to the second sealing element (304) to apply the force on the second sealing element (304) to translate the second element in the distal direction.

7. The injection system of any one of claims 1 to 3, wherein the drive assembly (16) comprises a first driver configured to translate the syringe barrel (302) relative to the support platform (12) and a second driver linked to the second sealing element (304) to translate the second sealing element (304) relative to the syringe barrel (302).

8. The injection system of any one of claims 1 to 3, wherein the one or more sensors comprise a first load cell configured to measure force on the syringe barrel (302) or the one or more sensors comprise a second load cell configured to measure force on the second sealing element (304).

9. The injection system of any one of claims 1 to 3, wherein the one or more sensors comprise one or more of a ta sensor, force sensor, strain sensor, position sensor or low rate sensor.

10. The injection system of any one of claims 1-9, wherein the controller is programmed to implement one or more feedback loops to monitor the first opposing force and the second opposing force.

11. The injection system of any one of claims 1-9, wherein the controller is programmed to implement one or more feedback loops to monitor a pre-insertion of the puncture element (15) into the tissue, the one or more feedback loops being configured to monitor an increase in force onto the puncture element (15), to detect a drop in force on the puncture element (15), and based on the drop, to cause an advancement of the puncture element (15) by a pre-determined distance to imbed the puncture element (15) into the tissue.

12. The injection system of any one of claims 1-9, wherein the controller is programmed to implement one or more feedback loops to monitor an advancement of the puncture element (15) through the tissue, the one or more feedback loops being configured to measure a load on the second sealing element (304) and detect a drop in the load once the puncture element (15) reaches the space in the tissue.

13. The injection system of any one of claims 1-9, wherein the controller is programmed to implement one or more feedback loops to monitor an injection of the injection agent into the space, the one or more feedback loops being configured to control a velocity or an advancement distance of the second sealing element (304).

14. The injection system of any one of claims 1-9, wherein the controller is programmed to cause a retraction of the puncture element (15) by a pre-determined distance when the one or more sensors detect a drop in load on the second sealing element (304) or the controller is programmed to control a stopping distance of the puncture element (15) as the puncture element (15) enters the space.

15. The injection system of any one of claims 1-9, wherein i) the tissue is conjunctiva and the space is subconjunctival space, ii) the tissue is sclera and the space is suprachoroidal space, iii) the tissue is sclera and choroid and the space is intravitreal space or iv) the tissue is cornea and the space is an anterior chamber of an eye.

## Patentansprüche

1. Injektionssystem (10), umfassend:
eine Injektionsbaugruppe (14), umfassend einen Spritzenzylinder (302), der ein Lumen zwischen einem proximalen Ende (302p) und einem distalen Ende (302d) definiert, und ein zweites Dichtungselement (304), das innerhalb des Lumens bewegbar angeordnet ist, um ein Injektionsmittel aus einer Injektionskammer, die in dem Spritzenzylinder (302) definiert ist, zu verabreichen, und ein Punktionselement (15), das dazu konfiguriert ist, das Injektionsmittel in einen Hohlraum in einem Gewebe eines Patienten abzugeben, wobei das Gewebe weniger durchlässig für das Injektionsmittel ist, als der Hohlraum;
eine Trägerplattform (12), die dazu konfiguriert ist, die Injektionsbaugruppe (14) zu tragen und die Injektionsbaugruppe (14) relativ zu einer Injektionsstelle zu verankern;
eine Antriebsbaugruppe (16), die dazu konfiguriert ist, die Injektionsbaugruppe (14) zu betreiben;
einen oder mehrere Sensoren, die dazu konfiguriert sind, eine oder mehrere Kräfte auf der Injektionsbaugruppe (14) zu überwachen; und
eine Steuerung, die mit dem einen oder den mehreren Sensoren kommuniziert, um Informationen über die eine oder die mehreren Kräfte auf dem Injektionssystem (10) zu empfangen, und die dazu konfiguriert ist, basierend auf den Informationen die Antriebsbaugruppe (16) derart zu betreiben, dass das Punktionselement (15) durch das Gewebe in Richtung des Hohlraums vorgeschoben wird, sodass das Injektionsmittel in der Injektionskammer verbleibt, bis das Punktionselement (15) die Injektionskammer mit dem Hohlraum fluidisch verbindet.

2. Injektionssystem nach Anspruch 1, wobei die Injektionsbaugruppe (14) ferner Folgendes umfasst:
ein erstes Dichtungselement (306), das innerhalb des Lumens distal zu dem zweiten Dichtungselement (304) bewegbar angeordnet ist, wobei das erste Dichtungselement (306) und das zweite Dichtungselement (304) eine Abdichtung mit dem Lumen bilden und zwischen einander die Injektionskammer definieren, wobei das Punktionselement (15) mit der Injektionskammer fluidisch kommuniziert, um das Injektionsmittel aus der Injektionskammer in den Hohlraum in dem Gewebe des Patienten abzugeben,
wobei das erste Dichtungselement (306) ein Nadelkolben ist und, wenn eine Kraft auf das zweite Dichtungselement (304) in einer distalen Richtung ausgeübt wird,
sich das erste Dichtungselement (306), als Reaktion auf eine erste Gegenkraft beim Vorschieben des Punktionselements (15) durch das Gewebe, in der distalen Richtung bewegt, um das Punktionselement (15) in der distalen Richtung vorzuschieben, ohne das Injektionsmittel durch das Punktionselement (15) zu befördern, und
das erste Dichtungselement (306), als Reaktion auf eine zweite Gegenkraft, wenn die Injektionskammer mit dem Hohlraum fluidisch verbunden ist, unbewegt bleibt und das Injektionsmittel von der Injektionskammer durch das Punktionselement (15) befördert wird.

3. Injektionssystem nach Anspruch 1, wobei:
die Injektionsbaugruppe (14) ein erstes Dichtungselement (306) umfasst, das innerhalb des Lumens bewegbar angeordnet ist, wobei das zweite Dichtungselement (304) distal zu dem ersten Dichtungselement (306) ist, um eine Injektionskammer zu definieren, und das Punktionselement (15) mit der Injektionskammer fluidisch verbunden ist;
das erste Dichtungselement (306) ein Nadelkolben ist;
die Antriebsbaugruppe (16) dazu konfiguriert ist, eines oder beides aus dem Spritzenzylinder (302) oder dem zweiten Dichtungselement (304) relativ zu der Trägerplattform (12) zu verschieben;
ein oder mehrere Sensoren dazu konfiguriert, eine oder mehrere Kräfte auf der Injektionsbaugruppe (14) zu überwachen; und
die Steuerung derart konfiguriert ist, dass, wenn die Antriebsbaugruppe (16) das zweite Dichtungselement (304) in einer distalen Richtung verschiebt,
sich das erste Dichtungselement (306), als Reaktion auf eine erste Gegenkraft beim Vorschieben des Punktionselements (15) durch das Gewebe, in der distalen Richtung bewegt, um das Punktionselement (15) in der distalen Richtung vorzuschieben, ohne das Injektionsmittel durch das Punktionselement (15) zu befördern, und
das erste Dichtungselement (306), als Reaktion auf eine zweite Gegenkraft, wenn die Injektionskammer mit dem Hohlraum fluidisch verbunden ist, unbewegt bleibt und das Injektionsmittel von der Injektionskammer durch das Punktionselement (15) befördert wird.

4. Injektionssystem nach einem der Ansprüche 1 bis 3, wobei die Antriebsbaugruppe (16) dazu konfiguriert ist, den Spritzenzylinder (302) und das zweite Dichtungselement (304) unabhängig voneinander relativ zu der Trägerplattform (12) zu verschieben.

5. Injektionssystem nach einem der Ansprüche 1 bis 3, wobei die Antriebsbaugruppe (16) mit dem zweiten Dichtungselement (304) gekoppelt ist, um die Kraft auf das zweite Dichtungselement (304) anzuwenden, um das zweite Element in der distalen Richtung zu verschieben.

6. Injektionssystem nach einem der Ansprüche 1 bis 3, wobei die Antriebsbaugruppe (16) einen Linearantrieb, der mit dem zweiten Dichtungselement (304) gekoppelt ist, umfasst, um die Kraft auf das zweite Dichtungselement (304) anzuwenden, um das zweite Element in der distalen Richtung zu verschieben.

7. Injektionssystem nach einem der Ansprüche 1 bis 3, wobei die Antriebsbaugruppe (16) einen ersten Antreiber, der dazu konfiguriert ist, den Spritzenzylinder (302) relativ zu der Trägerplattform (12) zu verschieben, und einen zweiten Antreiber, der mit dem zweiten Dichtungselement (304) gekoppelt ist, um das zweite Dichtungselement (304) relativ zu dem Spritzenzylinder (302) zu verschieben, umfasst.

8. Injektionssystem nach einem der Ansprüche 1 bis 3, wobei der eine oder die mehreren Sensoren eine erste Wägezelle umfassen, die dazu konfiguriert ist, Kraft auf dem Spritzenzylinder (302) zu messen, oder der eine oder die mehreren Sensoren eine zweite Wägezelle umfassen, die dazu konfiguriert ist, Kraft auf dem zweiten Dichtungselement (304) zu messen.

9. Injektionssystem nach einem der Ansprüche 1 bis 3, wobei der eine oder die mehreren Sensoren eines oder mehrere aus einem TA-Sensor, einem Kraftsensor, einem Dehnungssensor, einem Positionssensor oder einem Sensor für niedrige Raten umfassen.

10. Injektionssystem nach einem der Ansprüche 1-9, wobei die Steuerung dazu programmiert ist, eine oder mehrere Rückkopplungsschleifen zu implementieren, um die erste Gegenkraft und die zweite Gegenkraft zu überwachen.

11. Injektionssystem nach einem der Ansprüche 1-9, wobei die Steuerung dazu programmiert ist, eine oder mehrere Rückkopplungsschleifen zu implementieren, um eine Voreinführung des Punktionselements (15) in das Gewebe zu überwachen, wobei die eine oder die mehreren Rückkopplungsschleifen dazu konfiguriert sind, eine Zunahme an Kraft auf das Punktionselement (15) zu überwachen, um eine Abnahme an Kraft auf das Punktionselement (15) zu erkennen und, basierend auf der Abnahme, einen Vorschub des Punktionselements (15) um ein vorbestimmtes Maß zu verursachen, um das Punktionselement (15) in dem Gewebe einzubetten.

12. Injektionssystem nach einem der Ansprüche 1-9, wobei die Steuerung dazu programmiert ist, eine oder mehrere Rückkopplungsschleifen zu implementieren, um einen Vorschub des Punktionselements (15) durch das Gewebe zu überwachen, wobei die eine oder die mehreren Rückkopplungsschleifen dazu konfiguriert sind, eine Last auf dem zweiten Dichtungselement (304) zu messen und eine Abnahme der Last zu erkennen, sobald das Punktionselement (15) den Hohlraum in dem Gewebe erreicht.

13. Injektionssystem nach einem der Ansprüche 1-9, wobei die Steuerung dazu programmiert ist, eine oder mehrere Rückkopplungsschleifen zu implementieren, um eine Injektion des Injektionsmittels in den Hohlraum zu überwachen, wobei die eine oder die mehreren Rückkopplungsschleifen dazu konfiguriert sind, eine Geschwindigkeit oder ein Vorschubmaß des zweiten Dichtungselements (304) zu steuern.

14. Injektionssystem nach einem der Ansprüche 1-9, wobei die Steuerung dazu programmiert ist, einen Rückzug des Punktionselements (15) um ein vorbestimmtes Maß zu verursachen, wenn der eine oder die mehreren Sensoren eine Abnahme an Last auf dem zweiten Dichtungselement (304) erkennen, oder die Steuerung dazu programmiert ist, ein Anhaltemaß des Punktionselements (15) zu steuern, wenn das Punktionselement (15) in den Hohlraum eintritt.

15. Injektionssystem nach einem der Ansprüche 1-9, wobei i) das Gewebe Bindehaut ist und der Hohlraum ein Hohlraum unter der Bindehaut ist, ii) das Gewebe Lederhaut ist und der Hohlraum ein Hohlraum oberhalb der Aderhaut ist, iii) das Gewebe Lederhaut und Aderhaut ist und der Hohlraum ein intravitrealer Hohlraum ist oder iv) das Gewebe Hornhaut ist und der Hohlraum eine vordere Kammer eines Auges ist.

## Revendications

1. Système d'injection (10) comprenant :
un ensemble d'injection (14) comprenant un corps de seringue (302) définissant une lumière entre une extrémité proximale (302p) et une extrémité distale (302d) et un second élément d'étanchéité (304) disposé de manière mobile à l'intérieur de la lumière pour distribuer un agent d'injection à partir d'une chambre d'injection définie dans le corps de seringue (302), et un élément de ponction (15) configuré pour délivrer l'agent d'injection dans un espace au sein d'un tissu d'un patient, le tissu étant moins perméable à l'agent d'injection que l'espace ;
une plateforme de support (12) configurée pour supporter l'ensemble d'injection (14) et ancrer l'ensemble d'injection (14) par rapport à un site d'injection ;
un ensemble d'entraînement (16) configuré pour actionner l'ensemble d'injection (14) ;
un ou plusieurs capteurs configurés pour surveiller une ou plusieurs forces sur l'ensemble d'injection (14) ; et
un dispositif de commande en communication avec les un ou plusieurs capteurs pour recevoir des informations concernant les une ou plusieurs forces sur le système d'injection (10), et étant configuré pour, sur la base des informations, actionner l'ensemble d'entraînement (16) afin de faire avancer l'élément de ponction (15) à travers le tissu vers l'espace de sorte que l'agent d'injection reste dans la chambre d'injection jusqu'à ce que l'élément de ponction (15) relie de manière fluidique la chambre d'injection à l'espace.

2. Système d'injection selon la revendication 1, dans lequel l'ensemble d'injection (14) comprend en outre :
un premier élément d'étanchéité (306) disposé de manière mobile à l'intérieur de la lumière de façon distale au second élément d'étanchéité (304), dans lequel le premier élément d'étanchéité (306) et le second élément d'étanchéité (304) forment un joint avec la lumière et définissent la chambre d'injection entre ceux-ci, l'élément de ponction (15) étant en communication fluidique avec la chambre d'injection pour délivrer l'agent d'injection à partir de la chambre d'injection dans l'espace au sein du tissu du patient,
dans lequel le premier élément d'étanchéité (306) est un piston à aiguille et lorsqu'une force est appliquée sur le second élément d'étanchéité (304) dans une direction distale,
en réponse à une première force opposée lorsque l'élément de ponction (15) progresse à travers le tissu, le premier élément d'étanchéité (306) se déplace dans la direction distale pour faire progresser l'élément de ponction (15) dans la direction distale, sans acheminer l'agent d'injection à travers l'élément de ponction (15), et
en réponse à une seconde force opposée lorsque la chambre d'injection est reliée de manière fluidique à l'espace, le premier élément d'étanchéité (306) reste stationnaire et l'agent d'injection est acheminé à partir de la chambre d'injection à travers l'élément de ponction (15).

3. Système d'injection selon la revendication 1, dans lequel :
l'ensemble d'injection (14) comprend un premier élément d'étanchéité (306) disposé de manière mobile à l'intérieur de la lumière, le second élément d'étanchéité (304) étant distal par rapport au premier élément d'étanchéité (306) pour définir une chambre d'injection et l'élément de ponction (15) est relié de manière fluidique à la chambre d'injection ;
le premier élément d'étanchéité (306) est un piston à aiguille ;
l'ensemble d'entraînement (16) est configuré pour déplacer l'un ou les deux parmi le corps de seringue (302) ou le second élément d'étanchéité (304) par rapport à la plateforme de support (12) ;
un ou plusieurs capteurs configurés pour surveiller une ou plusieurs forces sur l'ensemble d'injection (14) ; et
le dispositif de commande est configuré de sorte que lorsque l'ensemble d'entraînement (16) déplace le second élément d'étanchéité (304) dans une direction distale,
en réponse à une première force opposée lorsque l'élément de ponction (15) progresse à travers le tissu, le premier élément d'étanchéité (306) se déplace dans la direction distale pour faire progresser l'élément de ponction (15) dans la direction distale, sans acheminer l'agent d'injection à travers l'élément de ponction (15), et
en réponse à une seconde force opposée lorsque la chambre d'injection est reliée de manière fluidique à l'espace, le premier élément d'étanchéité (306) reste stationnaire et l'agent d'injection est acheminé à partir de la chambre d'injection à travers l'élément de ponction (15).

4. Système d'injection selon l'une quelconque des revendications 1 à 3, dans lequel l'ensemble d'entraînement (16) est configuré pour déplacer indépendamment l'un de l'autre le corps de la seringue (302) et le second élément d'étanchéité (304) par rapport à la plateforme de support (12).

5. Système d'injection selon l'une quelconque des revendications 1 à 3, dans lequel l'ensemble d'entraînement (16) est lié au second élément d'étanchéité (304) pour appliquer la force sur le second élément d'étanchéité (304) afin de déplacer le second élément dans la direction distale.

6. Système d'injection selon l'une quelconque des revendications 1 à 3, dans lequel l'ensemble d'entraînement (16) comprend un actionneur linéaire lié au second élément d'étanchéité (304) pour appliquer la force sur le second élément d'étanchéité (304) afin de déplacer le second élément dans la direction distale.

7. Système d'injection selon l'une quelconque des revendications 1 à 3, dans lequel l'ensemble d'entraînement (16) comprend un premier élément d'entraînement configuré pour déplacer le corps de seringue (302) par rapport à la plateforme de support (12) et un second élément d'entraînement lié au second élément d'étanchéité (304) pour déplacer le second élément d'étanchéité (304) par rapport au corps de la seringue (302).

8. Système d'injection selon l'une quelconque des revendications 1 à 3, dans lequel les un ou plusieurs capteurs comprennent une première cellule de charge configurée pour mesurer une force sur le corps de seringue (302) ou les un ou plusieurs capteurs comprennent une seconde cellule de charge configurée pour mesurer une force sur le second élément d'étanchéité (304).

9. Système d'injection selon l'une quelconque des revendications 1 à 3, dans lequel les un ou plusieurs capteurs comprennent un ou plusieurs parmi un capteur de température, un capteur de force, un capteur de contrainte, un capteur de position ou un capteur de faible vitesse.

10. Système d'injection selon l'une quelconque des revendications 1 à 9, dans lequel le dispositif de commande est programmé pour mettre en œuvre une ou plusieurs boucles de rétroaction afin de surveiller la première force opposée et la seconde force opposée.

11. Système d'injection selon l'une quelconque des revendications 1 à 9, dans lequel le dispositif de commande est programmé pour mettre en œuvre une ou plusieurs boucles de rétroaction afin de surveiller une pré-insertion de l'élément de ponction (15) dans le tissu, les une ou plusieurs boucles de rétroaction étant configurées pour surveiller une augmentation de la force sur l'élément de ponction (15), pour détecter une chute de la force sur l'élément de ponction (15) et, sur la base de la chute, pour entraîner une progression de l'élément de ponction (15) d'une distance prédéterminée afin d'ancrer l'élément de ponction (15) dans le tissu.

12. Système d'injection selon l'une quelconque des revendications 1 à 9, dans lequel le dispositif de commande est programmé pour mettre en œuvre une ou plusieurs boucles de rétroaction afin de surveiller une progression de l'élément de ponction (15) à travers le tissu, les une ou plusieurs boucles de rétroaction étant configurées pour mesurer une charge sur le second élément d'étanchéité (304) et détecter une chute de la charge une fois que l'élément de ponction (15) atteint l'espace dans le tissu.

13. Système d'injection selon l'une quelconque des revendications 1 à 9, dans lequel le dispositif de commande est programmé pour mettre en œuvre une ou plusieurs boucles de rétroaction afin de surveiller une injection de l'agent d'injection dans l'espace, les une ou plusieurs boucles de rétroaction étant configurées pour commander une vitesse ou une distance de progression du second élément d'étanchéité (304).

14. Système d'injection selon l'une quelconque des revendications 1 à 9, dans lequel le dispositif de commande est programmé pour entraîner une rétraction de l'élément de ponction (15) d'une distance prédéterminée lorsque les un ou plusieurs capteurs détectent une chute de charge sur le second élément d'étanchéité (304) ou le dispositif de commande est programmé pour commander une distance d'arrêt de l'élément de ponction (15) lorsque l'élément de ponction (15) entre dans l'espace.

15. Système d'injection selon l'une quelconque des revendications 1 à 9, dans lequel i) le tissu est la conjonctive et l'espace est l'espace sous-conjonctival, ii) le tissu est la sclère et l'espace est l'espace suprachoroïdien, iii) le tissu est la sclère et la choroïde et l'espace est l'espace intravitréen ou iv) le tissu est la cornée et l'espace est la chambre antérieure d'un œil.
